# EUROPEAN PATENT SPECIFICATION

(11) **EP 1 663 381 B1**
(45) Date of publication and mention of the grant of the patent: **19.05.2010**
(21) Application number: 03817458.7
(22) Date of filing: 13.06.2003
(51) Int. Cl.: A61M 37/00, A61K 9/00, A61K 41/00

(54) **SONOPHORESIS SKIN CARE DEVICE**
SONOPHORESE-HAUTPFLEGEVORRICHTUNG
DISPOSITIF POUR SOINS DE LA PEAU PAR SONOPHORESE

(43) Date of publication of application: 07.06.2006
(73) Proprietor: THE PROCTER & GAMBLE COMPANY, Cincinnati, Ohio 45202 (US); Panasonic Electric Works Co., Ltd., Kadoma-shi Osaka (JP)
(72) Inventor: NUNOMURA, Mahito, Yawata, Kyoto 614-8297 (JP); OBA, Takafumi, Kuastsu, Shiga 525-0027 (JP); MATSUMURA, Yuko, Takatsuki, Osaka 569-1029 (JP); NONAKA, Gen, Higashinada-ku, Kobe, Hyogo 658-0015 (JP); TANAKA, Hidekazu, Higashinada-ku, Kobe, Hyogo 658-0072 (JP)
(74) Representative: Wilding, Richard Alan
(86) International application number: PCT/US2003/018828
(87) International publication number: WO 2005/004972

(56) References cited:
- WO-A-96/02276
- US-A- 4 372 296
- US-A- 4 787 888
- US-A- 5 383 848
- US-A- 5 722 397
- US-A1- 2001 041 856
- US-A1- 2002 055 702

## Description

### FIELD OF THE INVENTION

The present invention relates to a skin care device that comprises a composition and an ultrasound applying apparatus. The device provides improved penetration of skin active agents to the skin, while also being safe for the general consumer to use.

### BACKGROUND

Various treatment for the skin are proposed for delaying, minimizing or even eliminating skin hyperpigmentation (age spots, freckles, blotches, darkening, uneven tone, and the like), wrinkling and other chronical changes typically associated with skin aging or environmental damage to human skin. Such treatment range from application of specialty cosmetics such as packs and masks, oral intake of vitamins, to chemical peeling, laser surgery, photofacial, and others. Generally, it is believed that effective treatment requires more time, physical, and financial commitment. There is a high desire for a treatment which is effective, but is safe and reasonably priced such that the consumer can provide the treatment by himself/herself. PCT application WO 98/51255 teaches an ultrasound application device which has multiple safety features suitable for use by a layperson without the aid of a specialist. Such type of treatment is attractive to the general consumer, as the treatment can be provided at home by the consumer at his/her discretion.

The use of ultrasound to deliver agents transcutaneously, generally termed "sonophoresis" or "phonophoresis", is known in the art, for example in GB publication 1577551, PCT publication WO 88/00001, PCT publication WO 91/12772, PCT publication WO 94/08655, US patent 5267985, PCT publication WO 97/04832, US patent 5445611, PCT publication WO 97/40679, PCT publication WO 99/51295, US patent 6066123, Japanese patent publication A-11-335271, US publication 2002-55702, and PCT publication WO 00/21605. The depth of penetration into skin of ultrasound is inversely related to the frequency. Ultrasound at lower frequency is believed to provide effect towards deeply into the tissue, thus is effectively used for diagnosis, while higher frequency is believed to provide more effect towards the surface of the skin. PCT publication WO 88/00001 teaches the use of ultrasound at a frequency of no more than about 2.5 MHz for effectively delivering drugs to the circulatory system. To provide an effect to the epidermis of the skin, for example improved penetration of active ingredients that benefit to the epidermis of the skin, ultrasound at higher frequency is effective. Copending Japanese patent application 2002-012143 describes use of higher frequencies for delivery of skin active agents. A polymer gel network for cosmetic use is disclosed in PCT publication WO96/02276.

For providing a skin care treatment that is safe and effective for the general consumer, further improvement is desired. For example, aqueous gel carriers that have been proposed for use with ultrasound application have been unsatisfactory in effective delivery of skin active agents to the basale epidermidis, and further unsatisfactory in providing good aesthetic/sensory benefits to the skin compared to cosmetic products. In another example, the ultrasound application devices have been unsatisfactory in providing safety features that effectively operate even when applying ultrasound at higher frequencies.

Based on the foregoing, there is a need for a skin care device or method which provides safe and effective skin care treatment benefit via the combined use of skin active agent and ultrasound application. Specifically; there is a need for a composition which, when used in combination with an ultrasound applying apparatus, can effectively deliver the ultrasound to the skin, is stable, while providing smoothness and moisturization to the skin without leaving the skin feel sticky. Meanwhile, there is a need for an ultrasound application apparatus which has a control element for providing the frequency and intensity of ultrasound at a safe and effective level.

None of the existing art provides all of the advantages and benefits of the present invention.

### SUMMARY

The present invention is directed to a device for penetrating a skin active agent to the human body via the skin by the use of an ultrasound applying apparatus which applies ultrasound to the human body via the skin, as described in claim 1 :

These and other features, aspects, and advantages of the present invention will become evident to those skilled in the art from a reading of the present disclosure with the appended claims.

### BRIEF DESCRIPTION OF THE FIGURES

While the specification concludes with claims particularly pointing out and distinctly claiming the invention, it is believed that the present invention will be better understood from the following description of preferred, nonlimiting embodiments and representations taken in conjunction with the accompanying drawings in which:
FIG. 1 is a front sectional view of an ultrasound applying skin care device in accordance with a preferred embodiment of the present invention;
FIGS. 2A to 2C show improper use conditions of above device;
FIG. 3 is a block diagram of an electric circuitry of the above device;
FIG. 4 is a circuit diagram illustrating a driver circuit, a load detecting circuit, a motion detecting circuit of the above circuitry;
FIGS. 5A to 5F are waveform charts illustrating the operation of the load detecting circuit and the motion detecting circuit;
FIG. 6 is a circuit diagram illustrating a temperature sensing circuit of the above circuitry;
FIG. 7 is a flow chart illustrating the operations of the device; -
FIG. 8 is a top plan view of an applicator head of the above device;
FIG. 9 is a sectional view of the applicator head;
FIGS. 10 to 12 are partial views illustrating modified structure of the applicator head;
FIG. 13 is a schematic view illustrating a relation between the wavelength of the ultrasound of different frequencies and a combined vibration mass of the applicator head;
FIG. 14 is a graph illustrating, as a comparative purpose, electrically equivalent impedances of the vibration mass given under a normally loaded condition, an unloaded condition, and an abnormally loaded condition, respectively, without a structure of reducing a parasitic resonance;
FIG. 15 is a top plan view of the vibrator element;
FIG. 16 is a sectional view of the vibrator element;
FIG. 17 is a graph illustrating the electrically equivalent impedances of the vibration mass given under the normally loaded condition, the unloaded condition, and the abnormally loaded condition, respectively, for the combined vibration mass in accordance with the embodiment of the present invention;
FIGS. 18 and 19 are top and sectional views of the vibrator element in accordance with a modification of the above embodiment;
FIGS. 20 and 21 are top and sectional views of the vibrator element in accordance with another modification of the above embodiment;
FIGS. 22 and 23 are top views illustrating further modifications of the above embodiment;
FIGS. 24 and 25 are top and sectional views of the vibrator element in accordance with a still further modification of the above embodiment;
FIGS. 26 and 27 are top view illustrating further modifications of the above embodiment;
FIGS. 28 and 29 are top and sectional views of the vibrator element in accordance with a more modification of the above embodiment;
FIGS. 30 and 31 are sectional views illustrating modifications of the above embodiment;
FIGS. 32 and 33 are top and sectional views of the vibrator element in accordance with a further modification of the above embodiment;
FIGS. 34 and 35 are top and sectional views of the vibrator element in accordance with a more modification of the above embodiment.

### DETAILED DESCRIPTION

While the specification concludes with claims particularly pointing out and distinctly claiming the invention, it is believed that the present invention will be better understood from the following description.

Herein, "comprising" means that other elements which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

All ingredients such as actives and other ingredients useful herein may be categorized or described by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

### SKIN CARE DEVICE

The present device comprises a composition and an ultrasound applying apparatus, and provides skin care benefit in a safe and effective manner. The device provides improved penetration of skin active agents to the skin, while also being safe for the general consumer to use. The skin care benefit of the skin active agent contained in the composition is enhanced by the application of an ultrasound at a frequency of from about 3 MHz to about 10 MHz, thus the efficacy of treatment is greater than that compared to the independent use of the present composition.

In addition to the frequency, the intensity of the ultrasound is also important. Since the energy of higher frequency ultrasound is consumed at a shallower part of the skin, the heat generation in this area is greater. Thus, greater intensity generates greater heat, and skin disorders such as burns become a concern. On the other hand, a certain level of intensity is necessary for enhancing skin penetration of the skin benefit agent. In the present invention, the intensity of ultrasound is from about 0.1W/cm² to about 2W/cm² per surface area of the skin.

In the present device, the present composition is used as a medium for applying ultrasound to the skin by the present apparatus. Accordingly, when ultrasound is applied by the present apparatus, the present composition is existing in the space between the present apparatus and the skin, the amount of the present composition being sufficient for substantially filling such space, and for allowing the present apparatus to move along the surface of the skin. Preferably, the space between the present apparatus and the skin is devoid of air. Without being bound by theory, the penetration of the present composition is believed to be enhanced as a result of loosening of the intercellular lipid of the stratum corneum by the ultrasound application. Further, it is believed that the rheology of the present composition is particularly suitable for moving the present apparatus along the surface of the skin, while also effectively delivering the ultrasound, and maintaining a stable gel structure despite the vibration and heat emerged by the ultrasound application.

### (1) THE COMPOSITION

### (a) Skin Active Agent

The present composition comprises a safe and effective amount of a skin active agent. The term "skin active agent" as used herein, means an active ingredient which provides a cosmetic and/or therapeutic effect to the area of application on the skin, hair, or nails. The skin active agents useful herein include skin lightening agents, anti-acne agents, emollients, non-steroidal anti-inflammatory agents, topical anaesthetics, artificial tanning agents, antiseptics, anti-microbial and anti-fungal actives, skin soothing agents, sunscreening agents, skin barrier repair agents, anti-wrinkle agents, anti-skin atrophy actives, lipids, sebum inhibitors, sebum inhibitors, skin sensates, protease inhibitors, skin tightening agents, anti-itch agents, hair growth inhibitors, desquamation enzyme enhancers, anti-glycation agents, and mixtures thereof. In general, the present composition comprises from about 0.001% to about 30%, preferably from about 0.001% to about 10% of at least one skin active agent.

The type and amount of skin active agents are selected so that the inclusion of a specific agent does not affect the stability of the composition. For example, while water-soluble agents are preferable from a composition stability point of view, water-insoluble agents may also be included to the extent it can be dispersed with the viscosifying agent and or optional lower alkyl alcohol carrier, and thus does not affect the stability of the present composition. The term "water soluble" with regard to skin active agents herein, relate to compounds that are completely dissolved to make a transparent solution when dissolved in ample amount of water at ambient temperature.

Skin lightening agents useful herein refer to active ingredients that improve hyperpigmentation as compared to pre-treatment. Without being bound by theory, use of skin lightening agents for the present composition is particularly useful, as the frequency provided by the present apparatus effectively stimulate the epidermis, particularly the melanocyte region, wherein melanin is generated. The combined use of the skin lightening agent and ultrasonic wave application is believed to provide synergistic skin lightening benefit.

Useful skin lightening agents herein include ascorbic acid compounds, vitamin B₃ compounds, azelaic acid, butyl hydroxyanisole, gallic acid and its derivatives, glycyrrhizinic acid, hydroquinone, kojic acid, arbutin, mulberry extract, and mixtures thereof. Use of combinations of skin lightening agents are believed to be advantageous in that they may provide skin lightening benefit through different mechanisms. Preferably, the skin lightening agent comprises a water soluble skin lightening agent selected from ascorbic acid compounds, vitamin B₃ compounds, azelaic acid, gallic acid and its derivatives, hydroquinone, kojic acid, arbutin, mulberry extract, and mixtures thereof. In one preferred embodiment, a combination of ascorbic acid compounds and vitamin B₃ compounds are used.

Ascorbic acid compounds useful herein include, ascorbic acid per se in the L-form, ascorbic acid salt, and derivatives thereof. Ascorbic acid salts useful herein include, sodium, potassium, lithium, calcium, magnesium, barium, ammonium and protamine salts. Ascorbic acid derivatives useful herein includes, for example, esters of ascorbic acid, and ester salts of ascorbic acid. Particularly preferred ascorbic acid compounds include 2-o--D-glucopyranosyl-L-ascorbic acid, which is an ester of ascorbic acid and glucose and usually referred to as L-ascorbic acid 2-glucoside or ascorbyl glucoside, and its metal salts, and L-ascorbic acid phosphate ester salts such as sodium ascorbyl phosphate, potassium ascorbyl phosphate, magnesium ascorbyl phosphate, and calcium ascorbyl phosphate. Commercially available ascorbic compounds include: magnesium ascorbyl phosphate available from Showa Denko, 2-o-D-glucopyranosyl-L-ascorbic acid available from Hayashibara and sodium L-ascorbyl phosphate with tradename STAY C available from Roche.

Vitamin B₃ compounds useful herein include, for example, those having the formula: 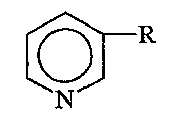 wherein R is -CONH₂ (*e.g*., niacinamide) or -CH₂OH (*e.g*., nicotinyl alcohol); derivatives thereof; and salts thereof. Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide. Preferred vitamin B₃ compounds are niacinamide and tocopherol nicotinate, and more preferred is niacinamide. In a preferred embodiment, the vitamin B₃ compound contains a limited amount of the salt form and is more preferably substantially free of salts of a vitamin B₃ compound. Preferably the vitamin B₃ compound contains less than about 50% of such salt, and is more preferably essentially free of the salt form. Commercially available vitamin B₃ compounds that are highly useful herein include niacinamide USP available from Reilly.

Other skin active agents useful herein include those selected from the group consisting of panthenol, tocopheryl nicotinate, benzoyl peroxide, 3-hydroxy benzoic acid, flavonoids (e.g., flavanone, chalcone), farnesol, phytantriol, glycolic acid, lactic acid, 4-hydroxy benzoic acid, acetyl salicylic acid, 2-hydroxybutanoic acid, 2-hydroxypentanoic acid, 2-hydroxyhexanoic acid, cis-retinoic acid, trans-retinoic acid, retinol, retinyl esters (e.g., retinyl propionate), phytic acid, N-acetyl-L-cysteine, lipoic acid, tocopherol and its esters (e.g., tocopheryl acetate), azelaic acid, arachidonic acid, tetracycline, ibuprofen, naproxen, ketoprofen, hydrocortisone, acetominophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, ketoconazole, neomycin sulfate, theophylline, and mixtures thereof. Preferred are those which are water soluble.

### (b) Viscosifying Agent

The present composition comprises a viscosifying agent that provides the composition a viscosity of from about 1,000mPas to about 1,000,000mPas, preferably from about 3,000mPas to about 100,000mPas. The polymers useful for providing the viscosifying agent herein are water soluble or water miscible polymers. The term "water soluble or water miscible" with regard to the viscosifying agents herein, relate to compounds that are dissolved to make a transparent solution when dissolved in ample amount of water with or without the aid of elevated temperature and/or mixing.

In one preferred embodiment, the viscosifying agent comprises a carboxylic acid/carboxylate copolymer and a cellulose derivative polymer. The combination of these polymers are believed to provide a composition that is transparent or translucent, while providing smoothness and moisturization to the skin without leaving the skin feel sticky. Other polymers compatible with the carboxylic acid/carboxylate copolymer and cellulose derivative polymer may also be included. In one highly preferred embodiment, the viscosifying agent is substantially made of only carboxylic acid/carboxylate copolymer and cellulose derivative polymer.

The present composition preferably comprises a carboxylic acid/carboxylate copolymer. The carboxylic acid/carboxylate copolymer keeps the composition relatively transparent and at a suitable viscosity without making the composition tacky or greasy upon use. Without being bound by theory, the carboxylic acid/carboxylate copolymer is also believed to provide a shear thinning property to the present composition. What is meant by shear thinning property is that a yield point exists within a typical shear stress applicable by the hand on the skin, and that the viscosity of the composition beyond the yield point significantly decreases to the extent such decrease is noticeable by the consumer.

Additionally, the carboxylic acid/carboxylate copolymer is capable of dispersing and stabilizing water insoluble components, such as water insoluble skin lightening agents in liquid form, in the present composition when such component is included.

The carboxylic acid/carboxylate copolymers herein are hydrophobically-modified crosslinked coplymers of carboxylic acid and alkyl carboxylate, and have an amphiphilic property. These carboxylic acid/carboxylate copolymers are obtained by copolymerizing 1) a carboxylic acid monomer such as acrylic acid, methacrylic acid, maleic acid, maleic anhydride, itaconic acid, fumaric acid, crotonic acid, or α-chloroacrylic acid, 2) a carboxylic ester having an alkyl chain of from 1 to about 30 carbons, and preferably 3) a crosslinking agent of the following formula: 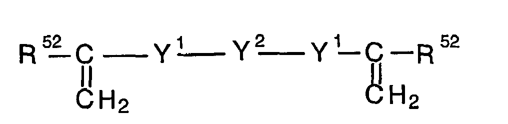 wherein R⁵² is a hydrogen or an alkyl group having from about 1 to about 30 carbons; Y¹ indepedently, is oxygen, CH₂O, COO, OCO, 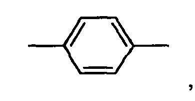 or 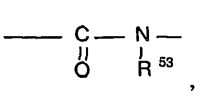 wherein R⁵³ is a hydrogen or an alkyl group having from about 1 to about 30 carbons; and Y² is selected from (CH₂)_{m"}, (CH₂CH₂O)_{m''}, or (CH₂CH₂CH₂O)_{m''} wherein m" is an integer of from 1 to about 30. It is believed that, because of the alkyl group contained in the copolymer, the carboxylic acid/carboxylate copolymers do not make the composition undesirably sticky.

Suitable carboxylic acid/carboxylate copolymers herein are acrylic acid/alkyl acrylate copolymers having the following formula: 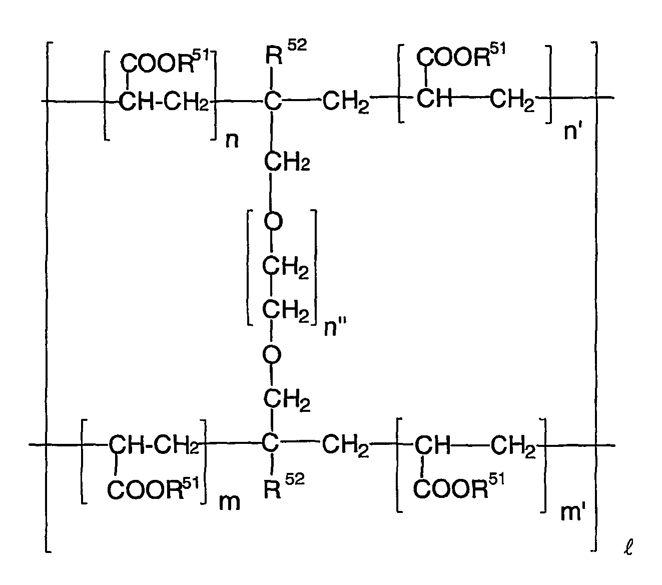 wherein R⁵¹, independently, is a hydrogen or an alkyl of 1 to 30 carbons wherein at least one of R⁵¹ is a hydrogen, R⁵² is as defined above, n, n', m and m' are integers in which n+n'+m+m' is from about 40 to about 100, n" is an integer of from 1 to about 30, and ℓ is defined so that the copolymer has a molecular weight of about 500,000 to about 3,000,000.

Commercially available carboxylic acid/carboxylate copolymers useful herein include: CTFA name Acrylates/C10-30 Alkyl Acrylate Crosspolymer having tradenames Pemulen TR-1, Pemulen TR-2, Carbopol 1342, Carbopol 1382, and Carbopol ETD 2020, all available from B. F. Goodrich Company.

Neutralizing agents may be included to neutralize the carboxylic acid/carboxylate copolymers herein. Nonlimiting examples of such neutralizing agents include sodium hydroxide, potassium hydroxide, ammonium hydroxide, monoethanolamine, diethanolamine, triethanolamine, diisopropanolamine, aminomethylpropanol, tromethamine, tetrahydroxypropyl ethylenediamine, and mixtures thereof.

The present composition preferably comprises a cellulose derivative polymer. Without being bound by theory, it is believed the controlled amount of cellulose derivative polymer in the composition provides improved moisturization and smoothness to the skin without giving an undesirable tacky or sticky feeling.

Cellulose derivative polymers useful herein include methylcellulose, ethylcellulose, hydroxyethylcellulose, hydroxyethyl ethylcellulose, hydroxypropyl methyl cellulose, nitrocellulose, sodium cellulose sulfate, sodium carboxymethylcellulose, crystalline cellulose, cellulose powder, and mixtures thereof. Particularly preferred are hydroxyethylcellulose carboxymethylcellulose, and mixtures thereof. Commercially available compounds that are highly useful herein include hydroxyethylcellulose with tradename Natrosol Hydroxyethylcellulose, and carboxymethylcellulose with tradename Aqualon Cellulose Gum, both available from Aqualon.

The compositions of the present invention may further comprise an additional water soluble polymer for the viscosifying agent.

Additional water soluble polymers useful herein include anionic polymers and nonionic polymers. Useful herein are vinyl polymers such as cross linked acrylic acid polymers with the CTFA name Carbomer, pullulan, mannan, scleroglucans, polyvinylpyrrolidone, polyvinyl alcohol, guar gum, hydroxypropyl guar gum, xanthan gum, acacia gum, arabia gum, tragacanth, galactan, carob gum, karaya gum, locust bean gum, carrageenin, pectin, amylopectin, agar, quince seed (Cydonia oblonga Mill), starch (rice, corn, potato, wheat), algae colloids (algae extract), microbiological polymers such as dextran, succinoglucan, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Polyalkylene glycols having a molecular weight of more than about 1000 are useful herein. Useful are those having the following general formula: 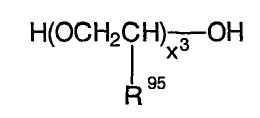 wherein R⁹⁵ is selected from the group consisting of H, methyl, and mixtures thereof. When R⁹⁵ is H, these materials are polymers of ethylene oxide, which are also known as polyethylene oxides, polyoxyethylenes, and polyethylene glycols. When R⁹⁵ is methyl, these materials are polymers of propylene oxide, which are also known as polypropylene oxides, polyoxypropylenes, and polypropylene glycols. When R⁹⁵ is methyl, it is also understood that various positional isomers of the resulting polymers can exist. In the above structure, x3 has an average value of from about 1500 to about 25,000, preferably from about 2500 to about 20,000, and more preferably from about 3500 to about 15,000. Other useful polymers include the polypropylene glycols and mixed polyethylene-polypropylene glycols, or polyoxyethylene-polyoxypropylene copolymer polymers,. Polyethylene glycol polymers useful herein are PEG-2M wherein R⁹⁵ equals H and x3 has an average value of about 2,000 (PEG-2M is also known as Polyox WSR^{®} N-10, which is available from Union Carbide and as PEG-2,000); PEG-5M wherein R⁹⁵ equals H and x3 has an average value of about 5,000 (PEG-5M is also known as Polyox WSR^{®} N-35 and Polyox WSR^{®} N-80, both available from Union Carbide and as PEG-5,000 and Polyethylene Glycol 300,000); PEG-7M wherein R⁹⁵ equals H and x3 has an average value of about 7,000 (PEG-7M is also known as Polyox WSR^{®} N-750 available from Union Carbide); PEG-9M wherein R⁹⁵ equals H and x3 has an average value of about 9,000 (PEG 9-M is also known as Polyox WSR^{®} N-3333 available from Union Carbide); and PEG-14 M wherein R⁹⁵ equals H and x3 has an average value of about 14,000 (PEG-14M is also known as Polyox WSR^{®} N-3000 available from Union Carbide).

Commercially available additional water soluble polymers highly useful herein include xanthan gum with tradename Keltrol series available from Kelco, Carbomers with tradenames Carbopol 934, Carbopol 940, Carbopol 950, Carbopol 980, and Carbopol 981, all available from B. F. Goodrich Company, acrylates/steareth-20 methacrylate copolymer with tradename ACRYSOL 22 available from Rohm and Hass, scleroglucan with tradename Clearogel SC11 available from Michel Mercier Products Inc. (NJ, USA), ethylene oxide and/or propylene oxide based polymers with tradenames CARBOWAX PEGs, POLYOX WASRs, and UCON FLUIDS, all supplied by Amerchol.

Additional water soluble polymers useful herein include amphoteric polymers. The amphoteric polymers useful herein are those including at least one cationic monomer and at least one anionic monomer; the cationic monomer being quaternary ammonium, preferably dialkyl diallyl ammonium chloride or carboxylamidoalkyl trialkyl ammonium chloride; and the anionic monomer being carboxylic acid. The amphoteric conditioning polymers herein may include nonionic monomers such as acrylamine, methacrylate, or ethacrylate.

Useful herein are polymers with the CTFA name Polyquaternium 22, Polyquaternium 39, and Polyquaternium 47. Such polymers are, for example, copolymers consisting of dimethyldiallyl ammonium chloride and acrylic acid, terpolymers consisting of dimethyldiallyl ammonium chloride and acrylamide, and terpolymers consisting of acrylic acid methacrylamidopropyl trimethylammonium chloride and methyl acrylate such as those of the following formula wherein the ratio of n⁶:n⁷:n⁸ is 45:45:10:

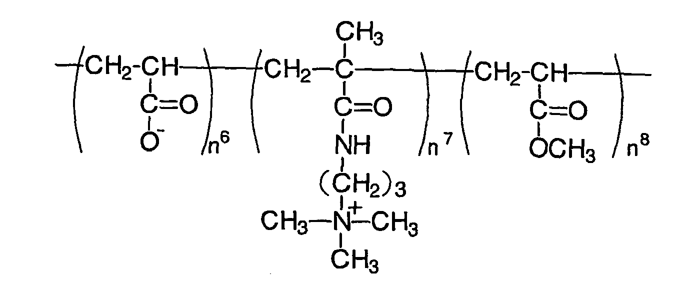

Highly preferred commercially available amphoteric polymers herein include Polyquaternium 22 with tradenames MERQUAT 280, MERQUAT 295, Polyquaternium 39 with tradenames MERQUAT PLUS 3330, MERQUAT PLUS 3331, and Polyquaternium 47 with tradenames MERQUAT 2001, MERQUAT 2001N, all available from Calgon Corporation.

Also useful herein are polymers resulting from the copolymerisation of a vinyl monomer carrying at least one carboxyl group, such as acrylic acid, methacrylic acid, maleic acid, itaconic acid, fumaric acid, crotonic acid, or alphachloroacrylic acid, and a basic monomer which is a substituted vinyl compound containing at least one basic nitrogen atom, such as dialkylaminoalkyl methacrylates and acrylates and dialkylaminoalkylmethacrylamides and acrylamides.

Also useful herein are polymers containing units derived from:
i) at least one monomer chosen from amongst acrylamides or methacrylamides substituted on the nitrogen by an alkyl radical,
ii) at least one acid comonomer containing one or more reactive carboxyl groups, and
iii) at least one basic comonomer, such as esters, with primary, secondary and tertiary amine substituents and quaternary ammonium substituents, of acrylic and methacrylic acids, and the product resulting from the quaternisation of dimethylaminoethyl methacrylate with dimethyl or diethyl sulfate.

The N-substituted acrylamides or methacrylamides which are most particularly preferred are the groups in which the alkyl radicals contain from 2 to 12 carbon atoms, especially N-ethylacrylamide, N-tert.-butylacrylamide, N-tert.-octylacrylamide, N-octylacrylamide, N-decylacrylamide and N-dodecylacrylamide and also the corresponding methacrylamides. The acid comonomers are chosen more particularly from amongst acrylic, methacrylic, crotonic, itaconic, maleic and fumaric acids and also the alkyl monoesters of maleic acid or fumaric acid in which alkyl has 1 to 4 carbon atoms.

The preferred basic comonomers are aminoethyl, butylaminoethyl, N,N'-dimethylaminoethyl and N-tert.-butylaminoethyl methacrylates.

### (c) Water Soluble Humectant

The composition of the present invention comprises from about 0.1% to about 30%, preferably from about 0.1% to about 10% of a water soluble humectant. Water soluble humectants useful herein include polyhydric alcohols such as butylene glycol (1,3 butanediol), pentylene glycol (1,2-pentanediol) glycerin, sorbitol, propylene glycol, hexylene glycol, ethoxylated glucose, 1,2-hexane diol, 1,2-pentane diol, hexanetriol, dipropylene glycol, erythritol, trehalose, diglycerin, xylitol, maltitol, maltose, glucose, fructose; and other water-soluble compounds such as sodium chondroitin sulfate, sodium hyaluronate, sodium adenosin phosphate, sodium lactate, pyrrolidone carbonate, glucosamine, cyclodextrin, and mixtures thereof. Also useful herein include water soluble alkoxylated nonionic polymers such as polyethylene glycols and polypropylene glycols having a molecular weight of up to about 1000 such as those with CTFA names PEG-200, PEG-400, PEG-600, PEG-1000, and mixtures thereof.

In one preferred embodiment, the water-soluble humectant is substantially selected only from the group consisting of butylene glycol, pentylene glycol, and mixtures thereof. These humectants provide moisturizing effect to the skin without significantly deteriorating the penetration of the skin lightening agents of the present invention.

Commercially available humectants herein include: butylenes glycol with tradename 1,3-Butylene glycol available from Celanese, pentylene glycol with tradename HYDROLITE-5 available from Dragoco, glycerin with tradenames STAR and SUPEROL available from The Procter & Gamble Company, CRODEROL GA7000 available from Croda Universal Ltd., PRECERIN series available from Unichema, and a same tradename as the chemical name available from NOF; propylene glycol with tradename LEXOL PG-865/855 available from Inolex, 1,2-PROPYLENE GLYCOL USP available from BASF; sorbitol with tradenames LIPONIC series available from Lipo, SORBO, ALEX, A-625, and A-641 available from ICI, and UNISWEET 70, UNISWEET CONC available from UPI; dipropylene glycol with the same tradename available from BASF; diglycerin with tradename DIGLYCEROL available from Solvay GmbH; xylitol with the same tradename available from Kyowa and Eizai; maltitol with tradename MALBIT available from Hayashibara, sodium chondroitin sulfate with the same tradename available from Freeman and Bioiberica, and with tradename ATOMERGIC SODIUM CHONDROITIN SULFATE available from Atomergic Chemetals; sodium hyaluronate available from Chisso Corp, the same with tradenames ACTIMOIST available from Active Organics, AVIAN SODIUM HYALURONATE series available from Intergen, HYALURONIC ACID Na available from Ichimaru Pharcos; sodium adenosin phophate with the same tradename available from Asahikasei, Kyowa, and Daiichi Seiyaku; sodium lactate with the same tradename available from Merck, Wako, and Showa Kako, cyclodextrin with tradenames CAVITRON available from American Maize, RHODOCAP series available from Rhone-Poulenc, and DEXPEARL available from Tomen; polyethylene glycols with the tradename CARBOWAX series available from Union Carbide, and a mixture of glyceryl polymethacrylate, propylene glycol and PVM/MA copolymer with tradename Lubrajel Oil available from Guardian Lab.

### (d) Aqueous Carrier

The compositions of the present invention comprise an aqueous carrier for providing a transparent or translucent composition, suitably called gels. The compositions of the present invention do not have a distinctive discontinuous phase, is not an emulsion, nor a liquid crystal. The present invention is substantially free of surfactants. When water-insoluble components are included in the present invention, such components are kept to an amount solubilizable/dispersible by, for example, carboxylic acid/carboxylate copolymers or lower alkyl alcohol.

The level and species of the carrier are selected according to the compatibility with other components, and other desired characteristic of the product. Carriers useful in the present invention include water and water solutions of lower alkyl alcohols. Lower alkyl alcohols useful herein are monohydric alcohols having 1 to 6 carbons, more preferably ethanol and isopropanol. Preferably, the present composition comprises at least about 70% water. Deionized water is preferably used. Water from natural sources including mineral cations can also be used, depending on the desired characteristic of the product.

The pH of the present composition is selected in view of the activity and stability of the skin lightening agents, and desired characteristic of the product. In one preferred embodiment where the skin lightening agent contains the combination of ascorbic acid compound and vitamin B₃ compound, the pH is preferably from about 5 to about 8. Buffers and other pH adjusting agents can be included to achieve the desirable pH.

### Free of Surfactant

The present composition is substantially free of surfactants. Preferably, there is less than about 1 % surfactant present, more preferably, there is less than about 0.5 % present, still preferably, there is no surfactant purposely added to the composition. What is meant by surfactants herein are any compounds that drastically decrease the surface tension and form micelles or reverse micelles above the critical micelle concentration when added to the composition. Anionic, amphoteric, zwitterionic, nonionic, and cationic surfactants that provide cleaning and lather upon application to the skin are included herein.

Anionic surfactants herein include ethoxylated alkyl sulphates, alkyl ethoxy carboxylates, alkyl glyceryl ether sulphonates, acyl sarcosinates, alkyl ethoxysulphosuccinates, alpha sulphonated fatty acids, their salts and/or their esters, ethoxylated alkyl phosphate esters, ethoxylated alkyl glyceryl ether sulfonates, paraffin sulfonates and alkoxy amide sulfonates, alkyl sulphates, and mixtures thereof.

Amphoteric surfactants herein include, cocoamphocarboxypropionate, cocoamphocarboxy propionic acid, cocoamphoacetate, cocoamphodiacetate (otherwise referred to as cocoamphocarboxyglycinate), sodium lauroamphoacetate (otherwise referred to as sodium lauroamphocarboxyglycinate).

Zwitterionic surfactants herein include, alkyl betaines and amido betaines.

Nonionic surfactants herein include, not only those that provide cleaning and lather, but also nonionic surfactants that primarily provide emulsification benefits. Such nonionic surfactants include condensation products of alkylene oxides which fatty acids, such as alkylene oxide esters of fatty acids, the condensation products of alkylene oxides with 2 moles of fatty acids, such as alkylene oxide diesters of fatty acids, the condensation products of alkylene oxides with fatty alcohols, examples of which include PEG 40 hydrogenated castor oil, steareth 2, isoceteth-20, and oleth-20. Other nonionic surfactants herein are the condensation products of alkylene oxides with both fatty acids and fatty alcohol, wherein the polyalkyene oxide portion is esterified on one end with a fatty acid and etherified on the other end with a fatty alcohol. Other nonionic surfactants herein are alkyl glucosides and alkyl polyglycosides, polyhydroxy fatty acid amide surfactants, alkoxylated sugar esters and polyesters, and fatty acid amides.

Cationic surfactants herein include: ammonium halide compounds, including those having hydrophilic substituents.

### Oily Component

In one preferred embodiment, the composition of the present invention contains oily components which are useful for providing moisturizing efficacy to the skin. The oily components herein are water-insoluble components, thus must be kept to an amount solubilizable/dispersible by, for example, carboxylic acid/carboxylate copolymers or lower alkyl alcohol. When included, the oily component is comprised at from about 0.1% to about 15%, preferably from about 0.5% to about 10%, of the entire composition.

A wide variety of suitable oil compounds are known and may be used herein and numerous examples can be found in Sagarin, Cosmetics, Science and Technology, 2nd Edition, Vol. 1, pp. 32-43 (1972). Nonlimiting examples of suitable oily components include C₁₋₃₀ alcohol esters of C₁₋₃₀ carboxylic acids and of C₂₋₃₀ dicarboxylic acids, hydrocarbon oils, Mono-, di- and tri- glycerides of C₁₋₃₀ carboxylic acids, silicone oils, mineral oil and petrolatums, vegetable oils and hydrogenated vegetable oils, animal fats and oils, silicone oils, aromatic oils, and mixtures thereof; preferably hydrocarbon oils, fatty acid esters, silicone oils, and mixtures thereof.

Hydrocarbon oils useful herein include these having from about 7 to about 40 carbons. Examples of these hydrocarbon materials include dodecane, isododecane, squalane, hydrogenated polyisobutylene, docosane (*i.e*., a C₂₂ hydrocarbon), hexadecane, and isohexadecane. Also useful are the C₇₋₄₀ isoparaffins, which are C₇₋₄₀ branched hydrocarbons. Preferred hydrocarbon oils are isohexadecane sold as Permethyl 101A available from Presperse, squalane, light paraffin, light isoparaffin, light liquid paraffin, light liquid isoparaffin (a commercially available hydrocarbon sold as Isoper G® by Exxon, Isoparaffin® 2028 by Idemitsu, Amoco Mineral Spirits® by Ashland).

Fatty acid esters useful herein include cetyl 2-ethylhexyl, isopropyl myristate, myristyl myristate, isopropyl palmitate, cholesterol; more preferably cetyl 2-ethylhexyl and myristyl myristate; and triglycerides such as caprylic/capric triglyceride, PEG-6 caprylic/capric triglyceride, and PEG-8 caprylic/capric triglyceride, Meadowfoam Seed Oil.

Silicone oils useful herein may be volatile, non-volatile, or a mixture of volatile and non-volatile silicones. The term "nonvolatile" as used in this context refers to those silicones that are liquid under ambient conditions and have a flash point (under one atmospheric of pressure) of or greater than about 100°C. The term "volatile" as used in this context refers to all other silicone oils. Suitable silicone oils can be selected from a wide variety of silicones spanning a broad range of volatilities and viscosities. While nonvolatile polysiloxanes are preferred, a small amount of volatile polysiloxanes may also be used. Nonlimiting examples of suitable silicones are disclosed in U.S. Patent No. 5,069,897, to Orr, issued December 3, 1991, which is incorporated by reference herein in its entirety. Examples of suitable silicone oils include polyalkylsiloxanes, cyclic polyalkylsiloxanes, and polyalkylarylsiloxanes. Commercially available polyalkylsiloxanes include the polydimethylsiloxanes, which are also known as dimethicones, examples of which include the Vicasil® series sold by General Electric Company and the Dow Corning® 200 series sold by Dow Corning Corporation. Suitable dimethicones include alkyl-substituted dimethicones such as cetyl dimethicone and lauryl dimethicone. Commercially available dimethiconols are typically sold as mixtures with dimethicone or cyclomethicone (*e.g*., Dow Corning® 1501 and 1503 fluids). Commercially available cyclic polyalkylsiloxanes include Dow Corning® 244 fluid, Dow Corning® 344 fluid, Dow Corning® 245, and Dow Corning® 345 fluid.

### Additional Components

The compositions herein may further contain other additional components, which may be selected by the artisan according to the desired characteristics of the final product and which are suitable for rendering the compositions more cosmetically or aesthetically acceptable or to provide them with additional usage benefits.

The present composition may further include skin benefit agents in addition to the skin lightening agents. The term "skin benefit agent" as used herein, means an active ingredient which provides a cosmetic and/or therapeutic effect to the area of application on the skin, hair, or nails. The additional skin benefit agents useful herein include anti-acne agents, emollients, non-steroidal anti-inflammatory agents, topical anaesthetics, artificial tanning agents, antiseptics, anti-microbial and anti-fungal actives, skin soothing agents, sunscreening agents, skin barrier repair agents, anti-wrinkle agents, anti-skin atrophy actives, lipids, sebum inhibitors, sebum inhibitors, skin sensates, protease inhibitors, skin tightening agents, anti-itch agents, hair growth inhibitors, desquamation enzyme enhancers, anti-glycation agents, and mixtures thereof.

The present composition may further include preservatives and preservative enhancers such as water-soluble or solubilizable preservatives including Germall 115, methyl, ethyl, propyl and butyl esters of hydroxybenzoic acid, benzyl alcohol, sodium metabisulfite, imidazolidinyl urea, EDTA and its salts, Bronopol (2-bromo-2-nitropropane-1,3-diol) and phenoxypropanol; antifoaming agents; binders; biological additives; bulking agents; coloring agents; perfumes, essential oils, and solubilizers thereof; other natural extracts; compounds which stimulate collagen production; yeast fermented filtrates, and others.

### (2) THE ULTRASOUND APPLYING APPARATUS

The ultrasound applying apparatus employed in the present invention includes a housing with an applicator head for applying the ultrasound to the user's skin, and a driver circuit that provides an electric pulse for actuating the applicator head to transmit the ultrasound to the skin. The applicator head is composed of a vibrator element generating the ultrasound, and a horn having a mounting face and a skin opposing face for use in contact with the skin. The horn carries the vibrator element on the mounting face to transmit the ultrasound to the skin. The vibrator element and the horn are integrated into a combined vibration mass that resonates with the electric pulse from the driver circuit, thereby transmitting resulting vibrations to the skin. The combined vibration mass gives a first electrically equivalent impedance when it is normally loaded by contact with the skin, and gives a second electrically equivalent impedance when it is unloaded. The apparatus includes a load detecting circuit which monitors whether the combined vibration mass give the first or second electrically equivalent impedance and provides a load detection signal only upon seeing the first electrically equivalent impedance. Also included in the apparatus is a control circuit which limits or stops the electric pulse when the load detection signal is not received within a predetermined time period. The combined vibration mass has a structure that restrains vibrations at a center portion of the vibration mass in order to reduce a parasitic resonance, thereby differentiating the first electrically equivalent impedance from the second electrically equivalent impedance. Thus, the load detecting circuit can successfully judge whether the applicator head is in contact with or out of contact from the skin, whereby the control circuit can be made reliable to limit the ultrasonic vibrations from being generated when the applicator head is unloaded. The control circuit is designed to receive the first electrically equivalent impedance and constitute the control element that varies the intensity of the ultrasound generated at the vibrator element in accordance with the magnitude of the first electrically equivalent impedance. As the first electrically equivalent impedance will vary depending upon a pressure at which the horn or the combined vibration mass is held against the user's skin, the device can vary the effect or the strength of the ultrasound being applied to the skin depending upon the pressure, thereby applying the ultrasound optimally to the user's skin for enhanced skin care result.

Preferably, the vibrator element is composed of a piezoelectric element in the form of a circular disc having flat upper and lower end faces provided respectively with upper and lower electrodes across which the electric pulse is applied. At least one of the upper electrode, the lower electrode, and the piezoelectric element has a center opening which is responsible for restraining the vibrations at the center of the combined vibration mass.

In addition to the center opening, at least one of the upper and lower electrodes may be dimensioned to have a diameter smaller than that of the piezoelectric element to leave the peripheral portion of the piezoelectric element uncovered also for reducing undesired vibrations around the periphery of the piezoelectric element.

Alternatively, at least one of the upper and lower electrodes is divided by at least one slit into a plurality of identical segments. The slit extends diametrically to leave the center and the diametrically extending band portion of the piezoelectric element uncovered for restraining the vibrations at the center of the vibration mass.

Instead of providing the diametrically extending slit, at least one of the upper and lower electrodes may be configured to have at least one slit that uncovers the center portion of the piezoelectric element for the same purpose of restraining the vibrations at the center of the vibration mass.

In combination with or separately from the provision of the center opening in at least one of the upper electrode, the lower electrode, and the piezoelectric element, the horn may be configured to have a center hole in the form of a through-hole or cavity for restraining the vibrations at the center of the vibration mass.

Further, instead of being formed with the center opening, the upper electrode may be covered with an elastic member that absorbs the vibrations at the center portion of the vibration mass for reducing the parasitic resonance.

Still further, the upper electrode may be covered on its center with a solder bulk for electrical connection of the upper electrode to a lead wire leading from the driver circuit. The solder bulk adds a weight to the center of the combined vibration mass for restraining the vibrations at the center portion thereof.

Further, the horn is preferred to be formed as an integral part thereof with a rim which surrounds the horn and which is adapted to secure the horn to the housing. Defined between the horn and the rim is a restrictor which restricts the ultrasound from propagating towards the rim, thereby concentrating the ultrasound to the horn for effectively transmitting the wave to the skin through the horn. The restrictor may be in the form of a cavity formed along the boundary between the horn and the rim.

Still further, the apparatus is preferred to include a motion detecting circuit which monitors whether the combined vibration mass is moving and provides a motion detection signal when the vibration mass is so moving. The control circuit is connected to receive the motion detection signal and controls the driver circuit to stop or limit the electric pulse when the motion detection signal is not continuous over a critical time duration even in the presence of the load detection signal being detected within the predetermined time period.

Now, the ultrasound applying apparatus utilized in the above device will be discussed in detail with reference to the attached drawings.

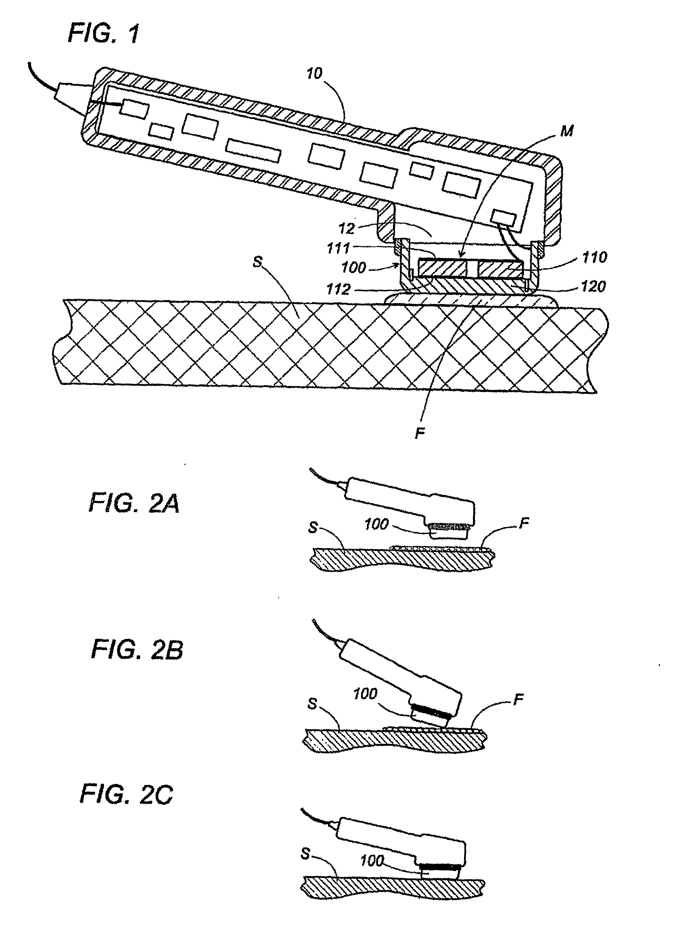

As shown in FIG. 1, the ultrasound applying apparatus a hand-held grip housing **10** provided at its one end thereof with an applicator head **100** which is adapted in use to contact with a user's skin for applying ultrasound thereto. The applicator head **100** is composed of a vibrator element **110** in the form of a piezoelectric element generating the ultrasound, and a horn **120** transmitting the ultrasound to the skin **S.** The piezoelectric element is shaped into a circular disc having a flat upper surface and a flat lower surface which are covered respectively with upper and lower electrodes **111** and **112** across which an electric pulse is applied for generating the ultrasound vibration. The vibrator element **110** and the horn **120** are integrated into a combined vibration mass **M** which is caused by the electric pulse to resonate for generating and applying the resonant ultrasound vibration to the skin **S.** Preferably, the apparatus is designed to generate the ultrasound having a frequency of 3 MHz to 10 MHz and transmitted to the skin at an intensity of 0.1 W/cm² to 2.0 W/cm². Prior to applying the ultrasound, the composition **F** is spread over the skin to occupy the space between the horn 120 and the skin S, as shown in FIG. 1.

As will be explained later in details, the apparatus is equipped with a safe-guard for limiting or stopping the ultrasonic vibrations being transmitted to the skin when the applicator head **100** is not in a normally loaded condition of FIG. 1, i.e., the applicator head is held in any one of improper conditions. As shown in FIGS. 2A to 2C, the improper conditions include an unloaded condition where the applicator head **100** is away from the skin (FIG. 2A), a partial contact condition where the applicator head **100** is placed only partially against the skin (FIG. 2B), and a direct contact condition where the applicator head **100** is placed against the skin without using the composition **F** at the interface therebetween (FIG. 2C).

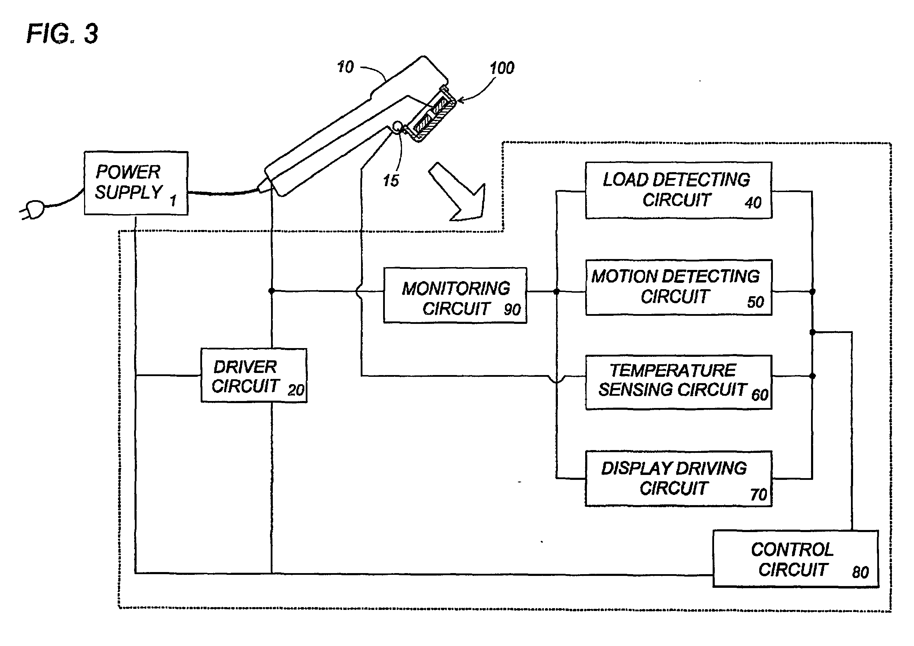

As shown in FIG. 3, the apparatus includes a driver circuit **20** for providing the electric pulse across the electrodes **111** and **112** of the piezoelectric element **110,** a load detecting circuit **40** for detection a load condition of the applicator head **100,** a motion detecting circuit **50** for detection of a motion of the applicator head **100,** a temperature sensing circuit **60** for sensing a temperature of the piezoelectric element **110,** a display driver circuit **170** for displaying operating conditions of the apparatus, and a control circuit **80** for control of the above circuits. The driver circuit **20** is energized by a power supply 1 accommodated within a separate power pack 2 for converting a commercial AC line voltage into a DC voltage. Also included in the apparatus is a monitoring circuit **90** for monitoring the ultrasound being generated and applied to the user's skin based upon an electrically equivalent impedance of the combined vibration mass **M.**

The apparatus **10** is designed to generate the ultrasound while the horn **120** is kept substantially in contact with the use's skin. For this purpose, the load detecting circuit **40** is provided to detect whether a suitable load is applied as a consequence of the horn **120** being in contact with the user's skin via the composition **F.** When the horn **120** is not in contact with the skin and fails to transmit the ultrasound successfully, the load detecting circuit **40** determines that the horn **120** or the vibration mass **M** is not loaded and restricts the generation of the ultrasound. The details of the load detection realized in the present invention will be discussed later. In use, it is desirable to move the applicator head **100,** i.e., the combination mass slowly across the skin when applying the ultrasound. Otherwise, when the applicator head **100** stays at a portion over a long period, there is a potential hazard of causing a cold burn in the skin. In view of this, the motion detecting circuit **50** is provided to enable the continuous ultrasound application when the applicator head **100** is moving at a suitable rate and otherwise disable or limit the ultrasound generation. In addition, the control circuit **80** includes a timer which stops generating the ultrasound after the apparatus is utilized over a preset time. That is, the timer will count a time only when the load detection signal from the load detecting circuit **40** indicates that the applicator head **100** is kept in the normal contact with the skin and when the motion detection signal from the motion detecting circuit **90** indicates that the applicator head **100** does not stay at a portion over a long time. The timer operates to continue generating the ultrasound over the preset time. Also, after the preset time is elapsed, the control circuit **80** gives an instruction to stop providing the electric power to the driver circuit **20,** ceasing the ultrasound generation.

When the vibration mass suffers from abnormal vibrations with an attendant temperature rise due to malfunction of the driver circuit **20** or the like, the temperature sensing circuit **60** is responsive to an output from a temperature sensor **15** located adjacent the horn **120** for providing an output indicative of abnormal temperature rise to the control circuit **80** which in turn responds to stop the driver circuit **20.**

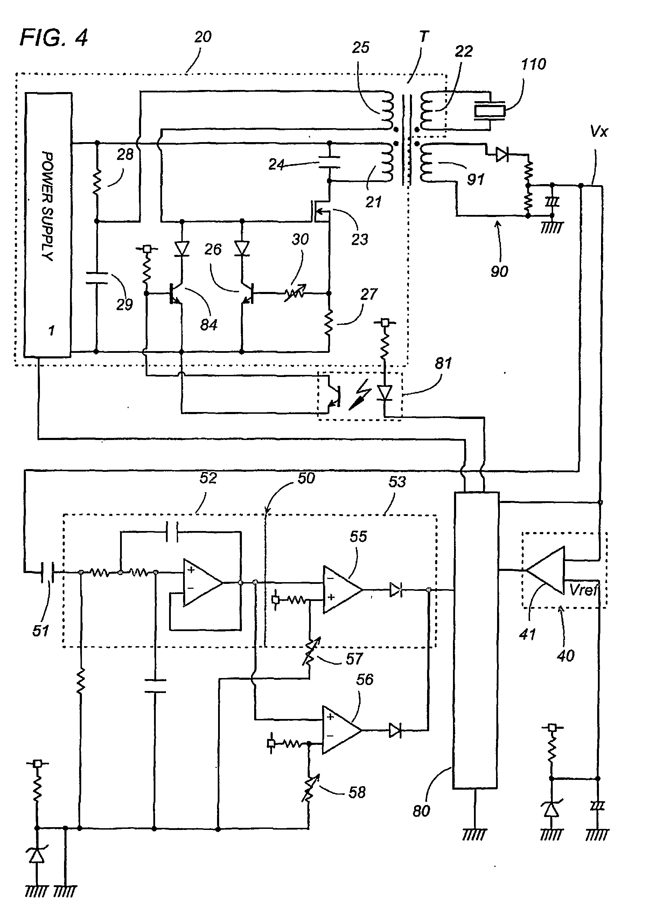

As shown in FIG. 4, the driver circuit **20** includes an inverter which converts DC voltage from the power supply **1** into an AC voltage. Provided at the output end of the inverter is a transformer T with a primary winding **21** and a secondary winding **22.** The primary winding **21** is connected in series with FET **23** and a current sensing resistor 27 across the power supply **1,** and is cooperative with a capacitor **24** to form a parallel resonant circuit which provides a resonant voltage across the primary winding **21** upon turning off of FET **23.** The piezoelectric element **110** is connected across the secondary winding **22** so as to effect the ultrasound vibrations by the AC voltage or the electric pulse induced at the secondary winding **22.** A feedback winding **25** is coupled to the primary winding **21** to feedback the output of the driver circuit to FET **23.** A bipolar transistor **26** is connected in a gate-emitter path of FET **23** for control of FET **23.** Connected across the power supply **1** is a series combination of a starting resistor **28** and a capacitor **29** of which connection is connected through the feedback winding **25** to a gate of FET **23** to give a bias thereto. When capacitor **29** is charged by the power supply **1** to develop a voltage reaching a threshold of FET **23,** FET becomes conductive to lower the drain voltage of FET **23.** At this time, the feedback winding **25** generates a feedback voltage applied to the gate of FET **23,** thereby increasing the current flowing through the FET. Subsequently when a voltage developed across current sensing resistor **27** reaches a predetermined level in correspondence to the increasing current through FET, transistor **26** becomes conductive to turn off FET **23.** Whereby, the resonant circuit of primary winding **21** and capacitor **24** becomes active to make a resonance. At the end of one cycle of the resonance, the feedback voltage induced at feedback winding **25** reaches a voltage of turning on the gate of FET **23,** thereby again making the FET conducive. The above operations are repeated to maintain the resonant voltage or the electric pulse so as to oscillate the piezoelectric element **110.** The frequency of the resonant circuit is set variable in the range of 3 MHz to 10 MHz.

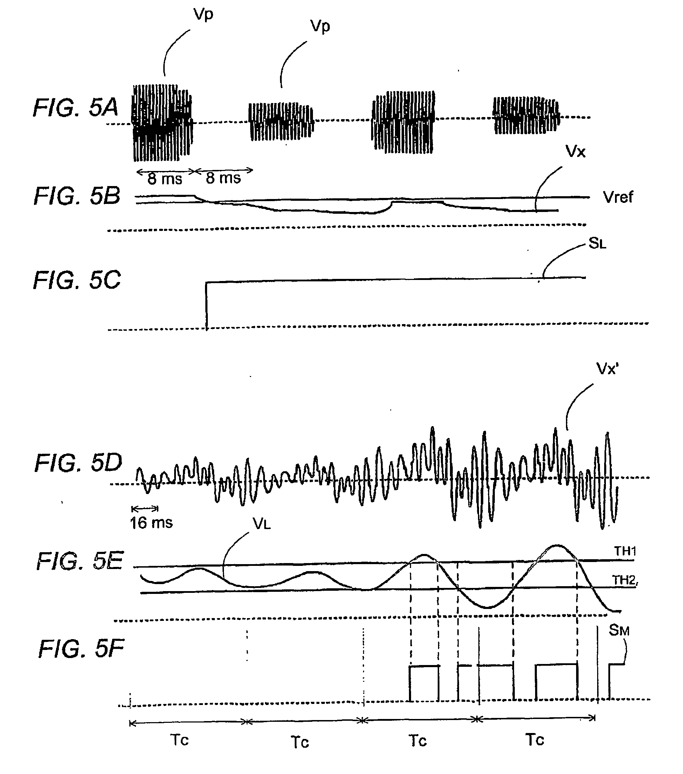

Connected between the base of transistor **26** and resistor **27** is a variable resistor **30** of which value is varied in order to adjust a timing of turning on transistor **26** for regulating the resonant frequency. It is noted in this connection that the resonant circuit is controlled by the control circuit **80** to give an intermittent oscillation having a rest period between adjacent pulse series Vp, as shown in FIGS. 5A and 5B.

The transformer **T** includes an auxiliary winding **91** which is cooperative with a rectifier circuit that rectifies the output of auxiliary winding **91** to constitute the monitoring circuit **90** which gives a monitoring output indicative of a condition of the ultrasound being applied to the user's skin. The monitoring output Vx includes low frequency components which are given as a result of moving the applicator head **100** and of which frequency is lower than that of the ultrasonic vibration. More precisely, the voltage appearing across auxiliary winding **91** includes low frequency components originating from a variation in electrically equivalent impedance of the combined vibration mass **M** upon contact with the load and originating from rubbing sounds appearing in response to the applicator head **100** moving across the skin of the user's skin, in addition to high frequency components indicative of the ultrasound vibrations. The monitoring output Vx is obtained by rectification of voltage appearing across auxiliary winding **91,** and is fed to the load detecting circuit **40** and the motion detecting circuit **50** for making the load detection and the motion detection.

The load detecting circuit **40** has a comparator **41** which compares the monitoring output Vx from the monitoring circuit **90** with a reference level Vref. The monitoring output Vx has a waveform pattern as shown in FIG. 5B. When monitoring output Vx becomes lower than the reference level Vref, the comparator **41** provides a H-level load detection signal SL to the control circuit **80** as indicative of that the applicator head **100** is kept in the normal contact with the user's skin. When the load detection signal SL is not acknowledged continuously over a predetermined time period, the control circuit **80** stops operating the driver circuit **20** or disables the power supply 1. In this embodiment, the load detection signal SL is generated when the monitoring output Vx is lower than the reference level Vref in consideration of that the resonant voltage is lowered by the presence of the load, i.e. the increased impedance of the combined vibration mass **M.**

In addition, the output Vx indicative of the impedance of the combined vibration mass **M** is fed also to the control circuit **80.** When the output Vx is equal to the reference level Vref or greater, the control circuit **80** operates to vary the output voltage of the power supply 1 a reverse proportion to the magnitude of the output Vx. That is, the combined vibration mass **M** is held against the user's skin at a greater pressure, the control circuit **80** acts to lower the intensity of the ultrasound being applied to the skin, and vice versa. With this result, the ultrasound can be adjusted depending upon the pressure at which the combined vibration mass **M** is held against the skin, thereby transmitting the ultrasound at an optimal intensity for enhanced skin care effect.

It is possible that resonant circuit of different configuration may vary the impedance characteristic of the combined vibration mass **M** in order to break the impedance matching with the resonant circuit, thereby causing the monitoring output to increase in the presence of the load. In this case, it is made to provide the load detection signal SL when the monitoring output Vx exceeds the reference level Vref. Also, it is equally possible to limit or reduce the ultrasound energy upon detection of the no-load condition.

Further, the monitoring output Vx is fed through a capacitor **51** to the motion detecting circuit **50** in the form of an output Vx', as shown in FIG. 5D. The motion detecting circuit 50 includes a low-pass filter **52** and a judging circuit **53.** The output Vx' is removed of high frequency components through the filter **52** to give a low frequency output VL free from the components not caused by the motion of the applicator head **100,** as shown in FIG. 5E. Thus obtained low frequency output VL is fed to two comparators **55** and **56** of the judging circuit 53 and compared respectively with individual thresholds TH1 and TH2 (TH1 > TH2) to provide to the control circuit **80** a H-level motion detection signal SM (shown in FIG. 5F) over a period in which the output VL is higher than the threshold TH1 or lower than the threshold TH2. TH1 and TH2 can be adjusted by variable resistors **57** and **58.** The control circuit **80** counts the time period of the H-level motion detection signal SM within a predetermined duration Tc (for example, 15 seconds) and determines that the applicator head **100** has moved suitably when the sum of the counted times within the duration Tc exceeds a predetermined reference. Otherwise, the control circuit **80** determines that no suitable motion has been made and provides a limit signal of limiting the driver circuit **20.** The driver circuit **20** includes a transistor **84** which is connected in parallel with transistor **26** across gate-source path of FET **23** and which is connected to the control circuit **80** through a photo-coupler **81.** Thus, upon receiving the limit signal from the control circuit **80,** the transistor **84** is turned on to thereby turn off FET **23** for disabling the driver circuit **20.** Although the limit signal acts to stop the driver circuit **20** in this embodiment, the present invention is not limited to this feature and may be arranged to control the driver circuit **20** or power supply 1 to reduce the ultrasonic vibration energy.

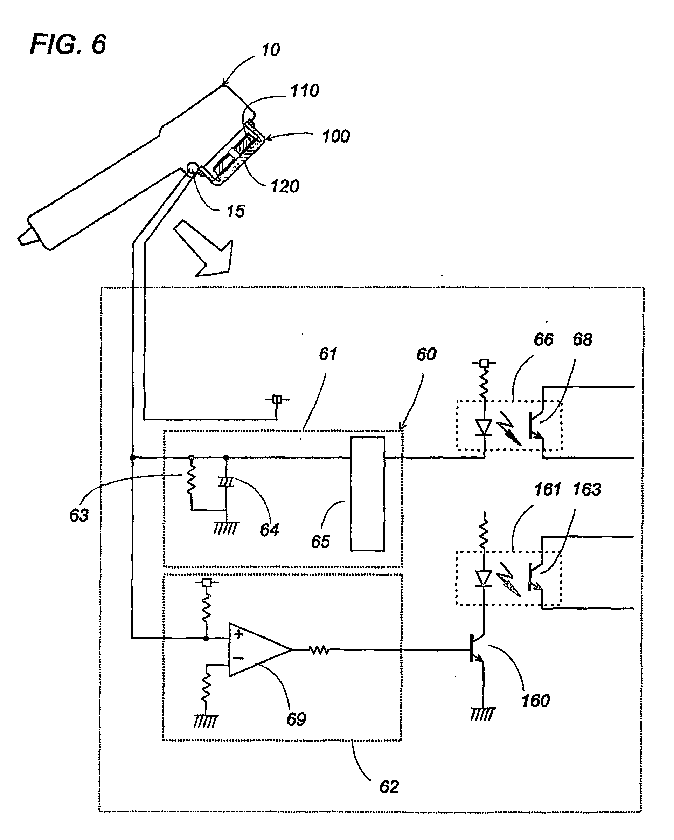

As shown in FIG. 6, the temperature sensing circuit **60** includes a first temperature sensing unit **61** and a second temperature sensing unit **62** both receiving an output from a thermistor **15** for temperature sensing. First temperature sensing unit **61** has a temperature controller **65** to which the output from thermistor **15** is fed through a resistor **63** and a capacitor **64.** When the temperature sensed at thermistor **15** is found to exceed a predetermined reference temperature, the temperature controller **65** issues a stop signal to the driver circuit **20** through a photo-coupler **66.** The photo-coupler **66** has a transistor **68** which is connected in a base-emitter path of the transistor **84,** so that the stop signal causes the transistor **84** to turn on for stopping the oscillation of the driver circuit **20.** A hysterics is given to the temperature control such that, after the temperature of the horn **120** sensed by thermistor **15** goes high above the reference temperature, the driver circuit **20** is enabled to resume the oscillation only after the sensed temperature goes below a temperature level which is lower than the reference temperature. When the sensed temperature goes below the temperature level, the temperature controller **65** responds not to issue the stop signal, thereby resuming the oscillation at the driver circuit **20.** The second temperature sensing unit **62** includes a comparator **69** which operates to turn on a transistor **160** when the temperature sensed at thermistor **15** exceeds a predetermined reference, thereby turning on a transistor **163** of a photo-coupler **161** and consequently disabling the power supply **1** connected to transistor **163.** The predetermined reference for the comparator **69** is set to be higher than the reference temperature of the temperature controller **65** for stopping the ultrasonic oscillation as a safeguard in response to the horn **120** being abnormally heated even if the temperature controller **65** made of a microprocessor should fail to operate.

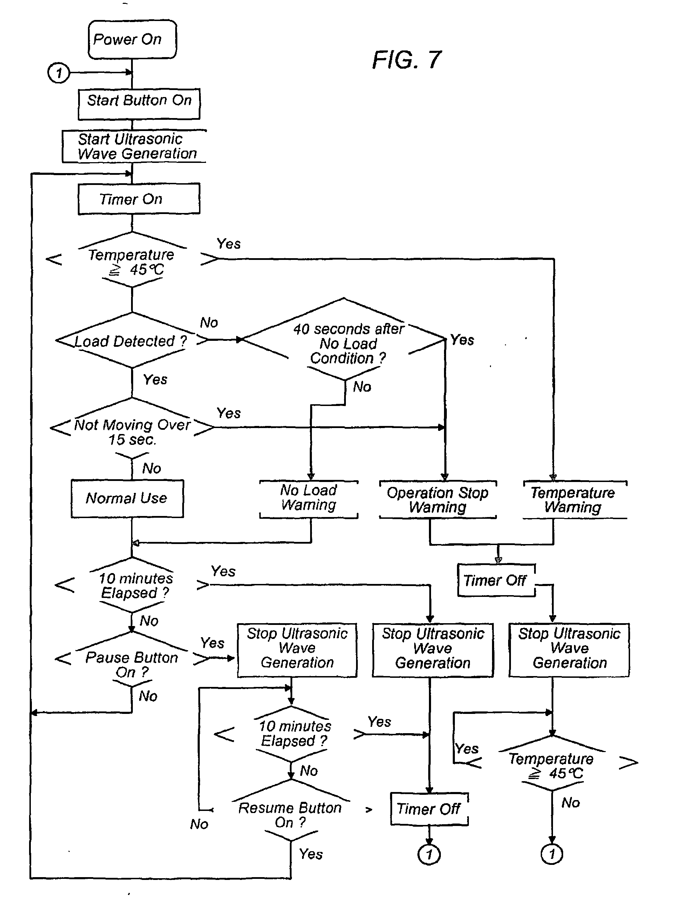

Operation of the ultrasonic apparatus is now explained with reference to FIG. 7. After turning on a power switch, pressing of a start button actuates the driver circuit **20,** causing the piezoelectric element **110** to start generating the ultrasound, and starting the timer. At this time, the temperature sensing is made for the horn **120** so that when the first temperature sensing unit **61** sees the temperature exceeding, for example, 45°, the display driving circuit **70** gives the temperature warning that the horn **120** is over-heated, and causing the timer as well as the driver circuit **20** to stop. When the sensed temperature is found to be less than 45°C at a step after starting the timer, the load detection is available, and subsequently the motion detection is available provided that the load detection signal is issued as indicative of that the applicator head **100** is loaded. When no load detection signal is issued, a no-load warning is displayed for a limited time period of 40 seconds, for example, prompting the user to make the applicator head **100** in contact with the skin. After elapse of 40 seconds with no load detection signal, a control is made to display a warning of stopping the operation and stop the timer and the ultrasound generation. The motion detection is made in the presence of the load detection signal so that, when the motion detection signal is issued within, for example, 15 seconds, a display of normal operation is made and a count-down instruction is given to the timer. After the elapse of a predetermined operation time, say, 10 minutes in this condition, the driver circuit is stopped. When a pause button is pressed within 10 minutes, the driver circuit is stopped but with the timer operating continuously to count down. When a restart button is pressed within the 10 minutes, the driver circuit resumes generating the ultrasound.

Although the above embodiment is so designed that the control circuit disables the driver circuit when no load or no motion is detected, the present invention is not limited to this feature and is designed to reduce the ultrasound energy upon such detection.

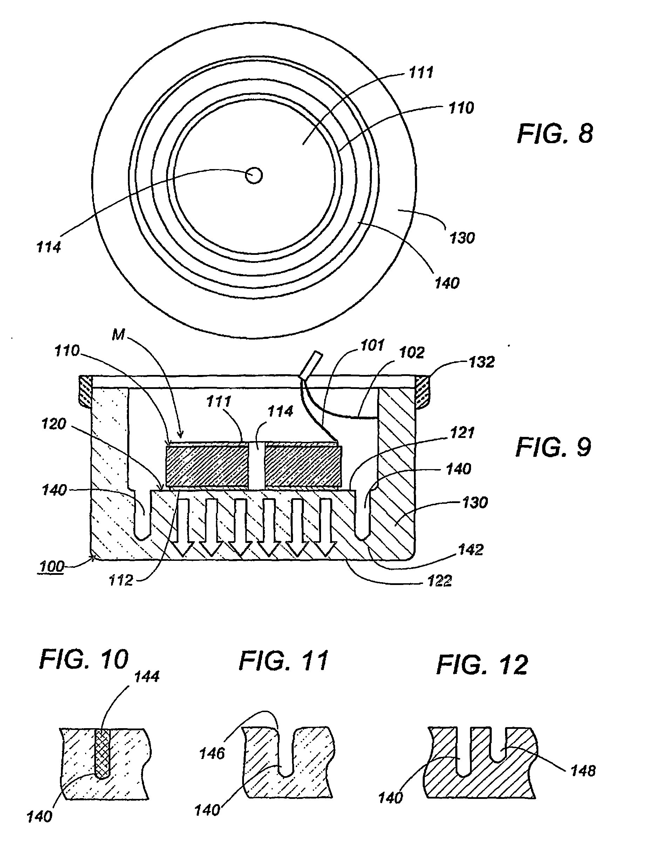

Now referring to FIGS. 8 and 9, the details of the applicator head **100,** i.e., the combination of the piezoelectric element **110** and the horn **120** will be discussed. The piezoelectric element **110** is made of a ceramic and shaped into the circular disk having a uniform thickness and being provided on its upper and lower faces respectively with the upper and lower electrodes **111** and **112.** The horn **120** is made of aluminum and is shaped into a circular disk having a surface area slightly larger than the piezoelectric element **110** and having a uniform thickness. The electric pulse from the driver circuit **20** is applied across the electrodes **111** and **112** by way of lead wires **101** and **102** respectively soldered to the upper electrode **111** and the horn **120,** as shown in FIG. 9. The horn **120** is formed as an integrally part thereof with a tubular rim 130 which surrounds the horn **120.** The rim **130** projects upwardly from the periphery of the horn **120** and is secured at its upper end to the housing **10** to support the applicator head **100** to the housing. The upper end of the rim **130** fits snugly into a mouth **12** of the housing **10** with an elastic damper ring **132** interposed therebetween. The horn **120** has a flat mounting face **121** for carrying thereon the piezoelectric element **110** in an intimate contact relation, and a flat skin opposing face **122** for contact with the skin through the composition **F** spread on the skin **S.** The piezoelectric element **110** is secured to the horn **120** such that they are integrated into the combined vibration mass **M** which resonates with the electric pulse from the driver circuit **20** to generate the ultrasound to be transmitted to the skin. A restrictor **140** in the form of a cavity extends between the horn **120** and the rim **130** in order to restrict the ultrasound vibrations from propagating towards the rim **130,** thereby concentrating the ultrasound effectively to the user's skin, as indicated by arrows in FIG. 9. That is, the cavity **140** acts to isolate the rim **130** substantially from the combined vibration mass **M** of the piezoelectric element **110** and the horn **120,** with regard to the ultrasound vibrations. As a result of forming the cavity **140,** a bridge **142** of reduced thickness remains for connection of the horn **120** and the rim **130.** The reduced thickness (t) of the bridge **142** is selected to be other than an integral multiple of one-fourth of the wavelength of the ultrasound (t ≠ n·λ/4, where n is an integer)) for effectively restricting the ultrasound vibrations from propagating towards the rim **130.** As shown in FIGS. 10 and 11, the cavity **140** may be filled with a suitable medium **144** for blocking the ultrasound vibrations, or may be finished with rounded edges **146.** Further, as shown in FIG. 12, an additional cavity **148** of different depth may be formed in a concentric relation to the cavity **140.**

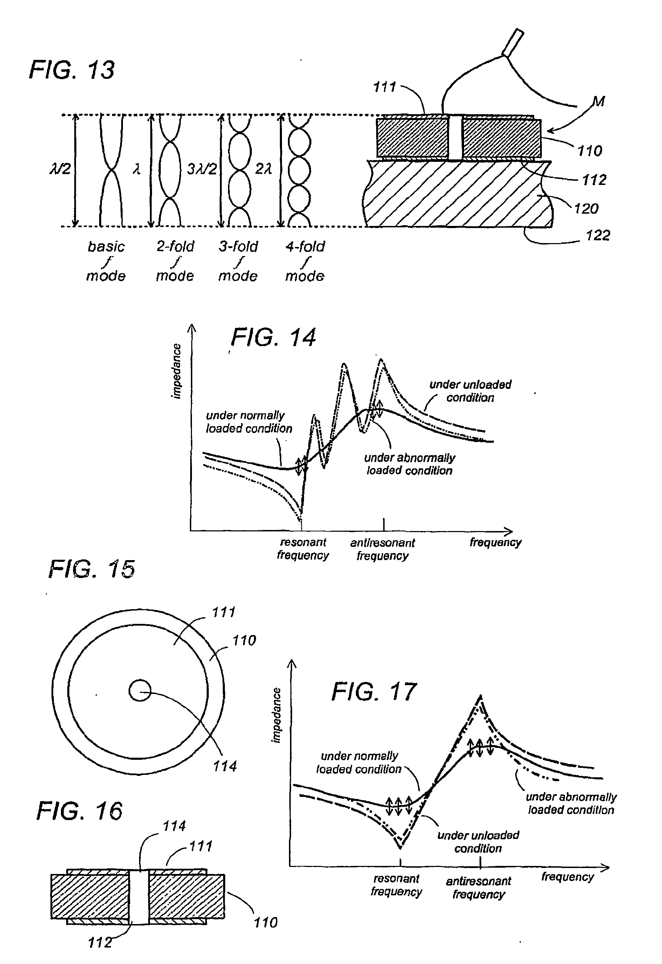

As shown in FIG. 13, the total thickness (T) of the combined vibration mass **M** of the piezoelectric element **110** and the horn **120** is selected to be half of wavelength (T=λ/2) of the ultrasound vibrating at a basic frequency of, for example, 3 MHz so that the combined vibration mass **M** can resonate also at the frequencies that are integral multiples of the basic frequency, for example, 2-fold, 3-fold, and 4-fold of the basic frequency, while forming antinodes at the skin opposing face **122** of the horn **120** and at the upper surface of the electrode **111,** as schematically seen in the figure.

In order to transmit the ultrasound power effectively to the skin at a minimum loss and also to discriminate the normally loaded condition from an abnormally loaded or the unloaded condition when actuating the combined vibration mass **M** around the resonant frequency, the piezoelectric element **110** is designed to have a structure that restrains vibrations at the center of the combined vibration mass **M** for reducing an undesired parasitic resonance which would otherwise makes the load detecting circuit **40** difficult to distinguish the normally loaded condition from the unloaded or abnormally loaded condition. That is, as shown in FIG. 14, the parasitic resonance brings about fluctuations which are superimposed on the electrically equivalent impedance curves as indicated by dotted lines with respect to varying frequency when the vibration mass **M** is under the unloaded condition or the abnormally loaded condition. With this result, it becomes practically difficult to distinguish the normally loaded condition from the unloaded or abnormally loaded condition on the basis of the impedance of the vibration mass **M** in the vicinity of the resonant or antiresonant frequencies. Consequently, it becomes hardly possible to extract the varying impedance indicating the contacting pressure of the vibration mass **M** within an admissible range as indicated by arrowed lines in the figure in the vicinity of the resonant or antiresonant frequencies, failing to vary the intensity of the ultrasound in accordance with the pressure at which the combination mass **M** is held against the user's skin, while the vibration mass **M** is in the normally loaded condition.

FIGS. 15 and 16 show one preferred structure for reducing the undesired parasitic resonance to such an extent that the load detecting circuit **40** can discriminate the normally loaded condition from the unloaded or abnormally loaded condition with reference to the electrically equivalent impedance of the combined vibration mass **M.** In this structure, a center opening **114** is formed to extend the center of the upper electrode **111,** the piezoelectric element **110,** and the lower electrode **112** for restraining the vibrations at the center of the piezoelectric element **110** and therefore the vibration mass **M.** With this result, the combined vibration mass **M** exhibits definite impedance characteristic curves in relation to the frequency under the unloaded or abnormally loaded condition, as indicated by dotted lines in FIG. 17, that can be well distinguished from the impedance curve that the vibration mass exhibits under the normally loaded condition, as indicated by solid line indicated in the same figure.

As apparent from FIG. 17, when subject to the unloaded or abnormally loaded condition, the vibration mass **M** can give the electrically equivalent impedance which are well distinctive from the impedance given under the normally loaded condition. By taking the advantage of the distinction, the load detecting circuit **40** can discriminate the abnormally loaded or unloaded condition successfully simply by monitoring the voltage reflecting the electrically equivalent impedance of the vibration mass **M,** as explained hereinbefore with reference to the monitoring circuit **90.** In this consequence, it becomes possible to extract the impedance varying with the contacting pressure of the vibration mass **M** within the admissible range as indicated by arrowed lines in the figure in the vicinity of the resonant or antiresonant frequencies. Whereby, it can be made to vary the intensity of the ultrasound in accordance with the pressure at which the combination mass **M** is held against the user's skin, as long as the vibration mass **M** is in the normally loaded condition.

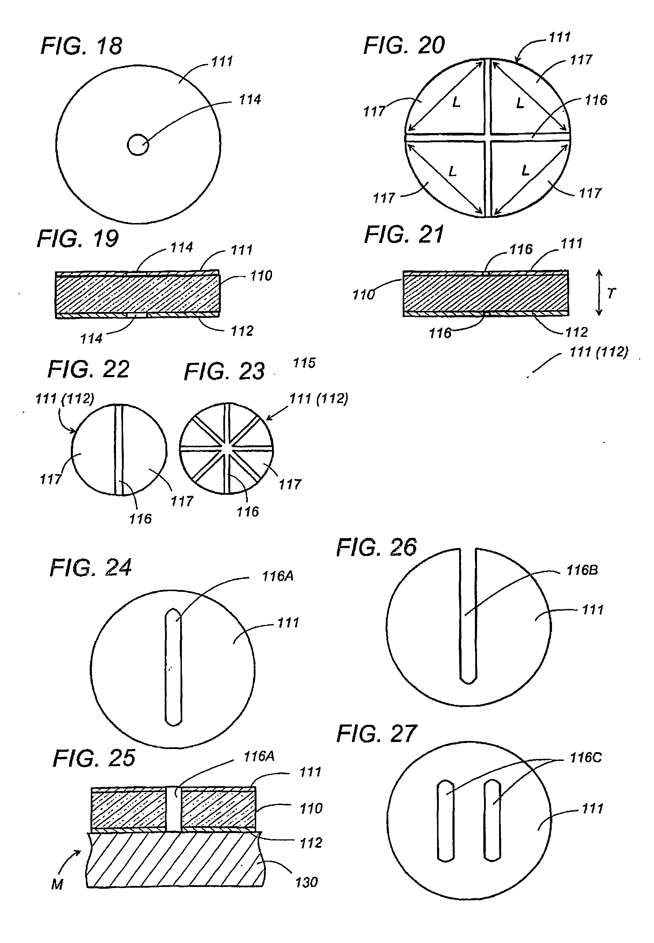

In combination with the center opening **114,** at least one of the upper and lower electrodes **111** and **112** may be shaped to have a diameter smaller than that of the piezoelectric element **110** to reduce the vibrations also at the periphery of the piezoelectric element and therefore the combined vibration mass **M** for further reducing the parasitic resonance. The center opening **114** may be formed in at lease one of the electrodes and the piezoelectric element, for example, as shown in FIGS. 18 and 19.

Further, as shown in FIGS. 20 and 21, the electrodes **111** and **112** may be divided by diametrically extending slits **116** into four identical segments or sectors **117.** The slits extend through the center of the electrodes to leave the center and the diametrically extending band portion of the piezoelectric element uncovered, thereby restraining the vibrations at the uncovered center and the band portions and therefore reducing the undesired parasitic resonance in order to realize the impedance characteristic of FIG. 17 as well.

Alternatively, one or both of the electrodes **111** and **112** may be divided into two or eight segments **117,** as shown in FIGS. 22 and 23 for the same purpose.

Further, it is possible to give a slit **116A** with closed ends also in the piezoelectric element **110** and the electrodes **111** and **112,** as shown in FIGS. 24 and 25, or to give a slit **116B** open at its one end, or to give parallel slits **116C,** as shown in FIGS. 26 and 27, in at least one of the electrodes and the piezoelectric element.

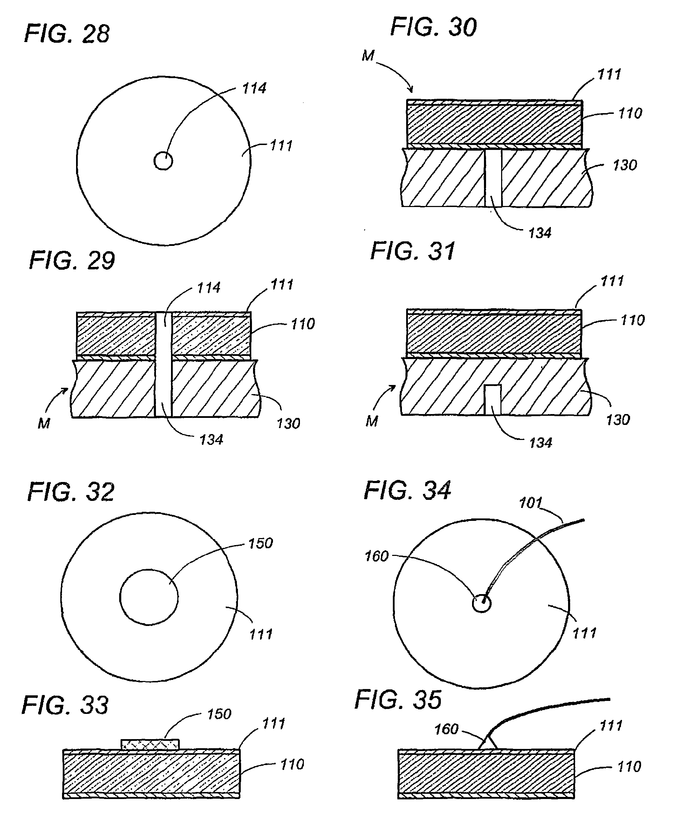

FIGS. 28 and 29 show a modification of the above embodiment in which a center hole **134** is formed in the horn **120** in alignment with the center opening **114** for further restraining the vibrations at the center of the combined vibration mass **M** and therefore reducing the undesired parasitic resonance to a large extent. The combination mass **M** may be provided only with the center hole **134** in the horn **120,** as shown in FIG. 30. In this instance, the center hole **134** may be in the form of a cavity, as shown in FIG. 31.

FIGS. 32 and 33 show an alternative structure in which an elastic member **150** is secured on the center of the upper electrode **111** for absorbing and therefore restraining the vibrations at the center of the piezoelectric element **110.** The elastic member **150** is preferably made of a silicone rubber. Instead of providing the elastic member **150,** it is equally possible to give a weight on the center of the electrode **111** for restraining the vibrations at the center of the piezoelectric element **110** and therefore reducing the undesired parasitic resonance at the center of the combined vibration mass **M.** The weight is given by a solder bulk **160** or land used for electrical connection of the electrode **111** to the lead wire **101** from the driver circuit **20.**

It is noted in this connection that the individual structures shown with reference to FIGS. 15, 16, 18 to 35 can be suitably combined for reducing the undesired parasitic resonance. Further, where the electrodes are concerned, it is possible that one of the electrodes can be given the above structure for reducing the vibrations at the center of the vibration mass, while leaving the other electrode to cover substantially entirely the corresponding face of the piezoelectric element **110.**

### EXAMPLES

The following examples further describe and demonstrate the preferred embodiments within the scope of the present invention. The examples are given solely for the purpose of illustration, and are not to be construed as limitations of the present invention since many variations thereof are possible without departing from its spirit and scope.

### EXAMPLES 1-6

The following compositions are formed by the process described herein:

### Compositions

| **Component** | Ex. 1 | Ex. 2 | Ex.3 | Ex. 4 | Ex. 5 | Ex.6 |
|---|---|---|---|---|---|---|
| Ascorbyl glucoside *1 | 2 | 2 | | 2 | 2 | 2 |
| Niacinamide *2 | 3.5 | 3.5 | 10 | 5 | 3.5 | 3.5 |
| Glycyrrhizinic acid *3 | | | 0.5 | | | |
| Acrylates/C10-30 alkyl acrylate crosspolymer *4 | 0.65 | 1 | 1 | 1 | 0.5 | 0.5 |
| Hydroxyethylcellulose *5 | 0.2 | 0.2 | 0.1 | 0.3 | | 0.5 |
| Carboxymethylcellulose *6 | | | | | 0.5 | 0.5 |
| Butylene glycol *7 | | 3 | 3 | 10 | 3 | 3 |
| Pentylene glycol *8 | 4 | 5 | | | | |
| Dimethicone/Dimethiconol *9 | 2 | 1.5 | | | | |
| Isohexadecane *10 | | | | 10 | | |
| Benzyl alcohol | | 0.2 | 0.2 | | | |
| Methylparaben | 0.15 | 0.15 | 0.15 | 0.15 | 0.15 | 0.15 |
| DisodiumEDTA | 0.1 | 0.1 | 0.1 | 0.1 | 0.1 | 0.1 |
| Sodium benzoate | 0.05 | 0.05 | 0.05 | 0.05 | 0.05 | 0.05 |
| Sodium metabisulfite | 0.03 | 0.03 | 0.03 | 0.03 | 0.03 | 0.03 |
| Ethanol | 10 | | | 10 | 10 | 10 |
| Sodium hydroxide | 0.2 | 0.3 | 0.3 | 0.3 | 0.2 | 0.2 |
| Deionized Water | To make total 100% | | | | | |

### Definitions of Components

* 1 Ascorbyl glucoside: Ascorbic Acid 2-Glucoside available from Hayashibara
* 2 Niacinamide: Niacinamide USP available from Reilly
* 3 Glycyrrhizinic acid: Glycyrrhizinic acid available from Maruzen
* 4 Acrylates/C10-30 alkyl acrylate crosspolymer: Pemulen TR-2 available from B. F. Goodrich Company
* 5 Hydroxyethylcellulose: Natrosol Hydroxyethylcellulose available from Aqualon
* 6 Carboxymethylcellulose: Aqualon Cellulose Gum available from Aqualon
* 7 Butylene glycol: 1,3-Butylene Glycol available from Celanese
* 8 Pentylene glycol: Hydrolite-5 available from Dragoco
* 9 Dimethicone/Dimethiconol: DC-1503 available from Dow Corning
* 10 Isohexadecane: Permethyl 101A from Presperse

### Method of Preparation

The skin care compositions of Examples 1 - 6 can be prepared by any conventional method known in the art. Suitably, the compositions are prepared as follows:
Cellulose derivative polymer, and acrylates/C10-30 alkyl acrylate crosspolymer, as included, are added in water and mixed to dissolve. The obtained mixture is heated to at least about 70°C, and butylene glycol, pentylene glycol, Dimethicone/Dimethiconol, isohexadecane, benzyl alcohol, methylparaben, disodium EDTA, sodium metabisulfite, and sodium benzoate, as included in the composition, are added. The obtained mixture is cooled to no greater than about 40°C, and ascorbyl glucoside, niacinamide, glycyrrhizinic acid, and ethanol, as included, are added. The finally obtained mixture is neutralized with sodium hydroxide. All of the compositions have a pH of between 5 and 8.

These embodiments represented by the previous figures and examples are useful as skin care devices.

When ultrasound at a frequency of from about 3MHz to about 10MHz and intensity of from about 0.1W/cm² to about 2W/cm² is applied by the present apparatus utilizing any of the present composition of Examples 1 - 6 as a medium, the composition assisted in moving the device along the surface of the skin, while also effectively delivering the ultrasounds, and retaining a stable gel structure. Further, a daily usage of the device for at least 2 weeks provided significant skin lightening benefits compared to the use of the present composition without application of ultrasound.

## Claims

1. A device for penetrating a skin active agent to the human body via the skin by the use of an ultrasound applying apparatus which applies ultrasound to the human body via the skin, comprising:
(1) a composition comprising:
(a) from 0.001% to 30% of the skin active agent;
(b) a viscosifying agent that provides the composition a viscosity of from 1,000 mPas to 1,000,000 mPas;
(c) from 0.1% to 30% of a water-soluble humectant ; and
(d) an aqueous carrier; wherein the composition is substantially free of surfactants; and
(2) the ultrasound applying apparatus comprising:
(e) an applicator head (100) for applying to the skin ultrasound at a frequency of from about 3 MHz to about 10 MHz and an intensity of from about 0.1 W/cm² to about 2 W/cm² ; and
(f) a control circuit (80) for controlling application conditions of the application element
wherein said ultrasonic applying apparatus further comprises : a housing (10) provided with the applicator head (100) which applies ultrasound to a user's skin; and a driver circuit (20) which gives an electric pulse for actuating said applicator head to generate the ultrasound; said applicator head comprising: a vibrator element (110) generating the ultrasound, and a horn (120) having a mounting face (121) and a skin opposing face (122) which is adapted in use to come into contact with the skin, said born carrying said vibrator on said mounting face to transmit said ultrasound to the skin through said skin opposing face, said vibrator element and said horn being integrated into a combined vibration mass which resonates with the electric pulse of a resonant frequency from said driver circuit to generate the ultrasound, said combination vibration mass defining said application element that gives a first electrically equivalent impedance when it is normally loaded by contact with the skin, and gives a second electrically equivalent impedance when it is unloaded, a load detecting circuit (40) which is connected to monitor whether said combined vibration mass gives the first or second electrically equivalent impedance and provides a load detection signal only upon seeing said first electrically equivalent impedance the control circuit (80) being adapted to limit or stop the electric pulse when the load detection signal is not received within a predetermined time period, said combined vibration mass having a structure that restrains vibrations at a portion of said combined vibration mass to reduce a parasitic resonance, thereby differentiating said first electrically equivalent impedance from said second electrically equivalent impedance for discrimination therebetween, said control circuit constituting said control element that receives said first electrically equivalent impedance in order to vary the intensity of the ultrasound generated at said vibrator element in accordance with the magnitude of said first electrically equivalent impedance.

2. The device of Claim 1 wherein the skin active agent is selected from ascorbic acid compounds, vitamin B3 compounds, and mixtures thereof.

3. The device of Claim 1 wherein the viscosifying agent comprises a carboxylicacid/carboxylate copolymer; and a cellulose derivative polymer.

4. The device of Claim 3 wherein the viscosifying agent provides a viscosity of from 3,000 mPas to 100,000 mPas.

5. The device of Claim 1 wherein the water-soluble humectant is selected from the group consisting of butylenes glycol, pentylene glycol, and mixtures thereof.

6. The device of Claim 1 wherein the composition comprises at least 70% water.

7. The device of Claim 1 wherein the composition further comprises from 0. 1% to 15% of an oily component.

8. The device of Claim 7 wherein the oily component is selected from the group consisting of hydrocarbon oils, fatty acid esters, silicone oils, and mixtures thereof.

9. The device as set forth in any of claims 1 to 8, wherein said vibrator element comprises a piezoelectric element (110) in the form of a circular disc having flat upper and lower end faces, and upper and lower electrodes (111, 112) respectively deposited on said upper and lower end faces, said electric pulse being applied across said upper and lower electrodes.

10. The device as set forth in claim 9, wherein at least one of said upper electrode, said lower electrode, and said piezoelectric element has a center opening (114) to restrain the vibrations at the center of said combined vibration mass.

11. The device as set forth in claim 9, wherein each of said upper electrode, said lower electrode, and said piezoelectric element has a center opening to restrain the vibrations at the center of said combined vibration mass.

12. The device as set forth in claim 10, wherein at least one of said upper electrode and said electrode has a diameter smaller than that of said piezoelectric element to leave the peripheral portions of the corresponding end face of said piezoelectric element uncovered.

13. The device as set forth in claim 9, wherein at least one of said upper and lower electrodes is divided by at least one slit into a plurality of identical segments, said at least one slit (116) extending diametrically to leave the center and the diametrically extending band portion of said piezoelectric element uncovered.

14. The device as set forth in claim 9, wherein at least one of said upper and lower electrodes has at least one slit that uncovers the center portion of said piezoelectric element.

15. The device as set forth in claim 9, wherein said horn has a center hole for restraining the vibrations at the center of said combined vibration mass.

16. The device as set forth in claim 10, wherein said horn has a center hole (134) for restraining the vibrations at the center of said combined vibration mass.

17. The device as set forth in claim 9, wherein said upper electrode is covered on its center with an elastic member (150) absorbing the vibrations at the center of said combined vibration mass.

18. The device as set forth in claim 17, wherein said elastic member is a silicone rubber.

19. The device as set forth in claim 9, wherein said upper electrode of said piezoelectric element is covered on its center with a solder bulk (160) for electrical connection of the upper electrode to a lead wire (101) leading from said driver circuit, the solder bulk adding a weight to the center of the piezoelectric element.

20. The device as set forth in claim 1, wherein said horn is formed as an integral part thereof with a rim (130) which surrounds said horn and is connected to said housing, said horn and said rim defines therebetween a restrictor (140) which restricts the ultrasound vibrations from propagating towards said rim.

21. The ultrasound applying skin care device as set forth in claim 20 wherein said restrictor is defined by a cavity formed at the boundary between said horn and said rim.

22. The ultrasound applying skin care device as set forth in any of claims 1 to 8, further including: a motion detecting circuit (50) which monitors whether said combined vibration mass is moving and provides a motion detection signal when said vibration mass is so moving; said control circuit controlling said driver circuit to stop or limit said electric pulse when said load detection signal is not received within said predetermined time period or when said motion detection signal is not continuous over a critical time duration even in the presence of said load detection signal being detected within said time period.

## Patentansprüche

1. Gerät für das Eindringen eines hautaktiven Wirkstoffs in den menschlichen Körper über die Haut durch Verwendung eines ultraschallabgebenden Geräts, das Ultraschall über die Haut auf den menschlichen Körper abgibt, umfassend:
(1) eine Zusammensetzung, umfassend:
(a) von 0,001 % bis 30 % den hautaktiven Wirkstoff;
(b) ein Verdickungsmittel, das der Zusammensetzung eine Viskosität von 1.000 mPas bis 1.000.000 mPas verleiht;
(c) von 0,1 % bis 30 % ein wasserlösliches Feuchthaltemittel; und
(d) einen wässrigen Träger; wobei die Zusammensetzung im Wesentlichen frei von Tensiden ist; und
(2) das ultraschallabgebende Gerät, umfassend:
(e) einen Applikatorkopf (100) zum Abgeben von Ultraschall auf die Haut mit einer Frequenz von etwa 3 MHz bis etwa 10 MHz und einer Intensität von etwa 0,1 W/cm² bis etwa 2 W/cm²; und
(f) eine Steuerschaltung (80) zur Steuerung der Anwendungsbedingungen des Applikationselements,
wobei das ultraschallabgebende Gerät ferner Folgendes umfasst: ein Gehäuse (10), das mit dem Applikatorkopf (100) versehen ist, der Ultraschall auf die Haut eines Benutzers abgibt; und eine Treiberschaltung (20), die einen elektrischen Impuls zur Betätigung des Applikatorkopfes abgibt, um den Ultraschall zu erzeugen; wobei der Applikatorkopf Folgendes umfasst: ein Vibratorelement (110), das den Ultraschall erzeugt, und ein Horn (120) mit einer Befestigungsseite (121) und einer der Haut gegenüberliegenden Seite (122), die bei Gebrauch angepasst wird, um mit der Haut in Kontakt zu kommen, wobei das Horn den Vibrator auf der Befestigungsseite trägt, um den Ultraschall durch die der Haut gegenüberliegenden Seite auf die Haut zu übertragen, wobei das Vibratorelement und das Horn in einer kombinierten Vibrationsmasse integriert sind, die mit dem elektrischen Impuls einer Resonanzfrequenz der Treiberschaltung mitschwingt, um den Ultraschall zu erzeugen, wobei die Kombinationsvibrationsmasse das Applikationselement bestimmt, das eine erste elektrisch gleichwertige Impedanz abgibt, wenn es durch Kontakt mit der Haut normal belastet ist, und eine zweite elektrisch gleichwertige Impedanz abgibt, wenn es unbelastet ist, eine Lastdetektionsschaltung (40), die angeschlossen ist, um zu überwachen, ob die kombinierte Vibrationsmasse die erste oder die zweite elektrisch gleichwertige Impedanz abgibt, und ein Lastdetektionssignal nur dann liefert, wenn die erste elektrisch gleichwertige Impedanz festgestellt wird, wobei die Steuerschaltung (80) so angepasst wird, dass der elektrische Impuls begrenzt oder beendet wird, wenn das Lastdetektionssignal nicht innerhalb eines vorbestimmten Zeitraums empfangen wird, wobei die kombinierte Vibrationsmasse eine Struktur aufweist, die Vibrationen in einem mittleren Abschnitt der kombinierten Vibrationsmasse zurückhält, um eine störende Resonanz zu reduzieren, wodurch die erste elektrisch gleichwertige Impedanz von der zweiten elektrisch gleichwertigen Impedanz zum Zwecke der Unterscheidung dazwischen differenziert wird, wobei die Steuerschaltung das Steuerelement darstellt, das die erste elektrisch gleichwertige Impedanz empfängt, um die Intensität des an dem Vibratorelement erzeugten Ultraschalls entsprechend der Größe der ersten elektrisch gleichwertigen Impedanz zu variieren.

2. Gerät nach Anspruch 1, wobei der hautaktive Wirkstoff ausgewählt ist aus Ascorbinsäureverbindungen, Vitamin-B3-Verbindungen und Mischungen davon.

3. Gerät nach Anspruch 1, wobei das Verdickungsmittel ein Carbonsäure/ Carboxylat-Copolymer und ein Cellulosederivat-Polymer umfasst.

4. Gerät nach Anspruch 3, wobei das Verdickungsmittel eine Viskosität von 3.000 mPas bis 100.000 mPas verleiht.

5. Gerät nach Anspruch 1, wobei das wasserlösliche Feuchthaltemittel ausgewählt ist aus der Gruppe bestehend aus Butylenglycol, Pentylenglycol und Mischungen davon.

6. Gerät nach Anspruch 1, wobei die Zusammensetzung zu mindestens 70 % Wasser umfasst.

7. Gerät nach Anspruch 1, wobei die Zusammensetzung ferner von 0,1% bis 15 % einen öligen Bestandteil umfasst.

8. Gerät nach Anspruch 7, wobei der ölige Bestandteil ausgewählt ist aus der Gruppe bestehend aus Kohlenwasserstoffölen, Fettsäureestern, Silikonölen und Mischungen davon.

9. Gerät nach einem der Ansprüche 1 bis 8, wobei das Vibratorelement ein piezoelektrisches Element (110) in Form einer runden Scheibe mit einer flachen oberen und unteren Endfläche und einer oberen und einer unteren Elektrode (111, 112), die an der oberen bzw. unteren Endfläche angeordnet sind, umfasst, wobei der elektrische Impuls über die obere und die untere Elektrode abgegeben wird.

10. Gerät nach Anspruch 9, wobei mindestens die obere Elektrode, die untere Elektrode und/oder das piezoelektrische Element eine zentrale Öffnung (114) aufweist/aufweisen, um die Vibrationen in der Mitte der kombinierten Vibrationsmasse zurückzuhalten.

11. Gerät nach Anspruch 9, wobei die obere Elektrode, die untere Elektrode und das piezoelektrische Element jeweils eine zentrale Öffnung aufweisen, um die Vibrationen in der Mitte der kombinierten Vibrationsmasse zurückzuhalten.

12. Gerät nach Anspruch 10, wobei mindestens die obere Elektrode und/oder die untere Elektrode einen Durchmesser aufweist/aufweisen, der kleiner ist als der des piezoelektrischen Elements, so dass die äußeren Abschnitte der entsprechenden Endfläche des piezoelektrischen Elements unbedeckt bleiben.

13. Gerät nach Anspruch 9, wobei mindestens die obere und/oder die untere Elektrode durch mindestens einen Schlitz in mehrere identische Segmente unterteilt ist/sind, wobei sich der mindestens eine Schlitz (116) diametral erstreckt, so dass die Mitte und der sich diametral erstreckende bandförmige Abschnitt des piezoelektrischen Elements unbedeckt bleiben.

14. Gerät nach Anspruch 9, wobei mindestens die obere und/oder untere Elektrode mindestens einen Schlitz aufweist/aufweisen, der den mittleren Abschnitt des piezoelektrischen Elements unbedeckt lässt.

15. Gerät nach Anspruch 9, wobei das Horn ein mittleres Loch aufweist, um die Vibrationen in der Mitte der kombinierten Vibrationsmasse zurückzuhalten.

16. Gerät nach Anspruch 10, wobei das Horn ein mittleres Loch (134) aufweist, um die Vibrationen in der Mitte der kombinierten Vibrationsmasse zurückzuhalten.

17. Gerät nach Anspruch 9, wobei die obere Elektrode in ihrer Mitte mit einem elastischen Element (150) bedeckt ist, das die Vibrationen in der Mitte der kombinierten Vibrationsmasse absorbiert.

18. Gerät nach Anspruch 17, wobei das elastische Element ein Silikongummi ist.

19. Gerät nach Anspruch 9, wobei die obere Elektrode des piezoelektrischen Elements in ihrer Mitte mit einer Lötmasse (160) bedeckt ist, um die obere Elektrode elektrisch mit einem aus der Treiberschaltung kommenden Zuleitungsdraht (101) zu verbinden, wobei die Lötmasse der Mitte des piezoelektrischen Elements ein Gewicht hinzufügt.

20. Gerät nach Anspruch 1, wobei das Horn als einstückiger Teil davon ausgebildet ist, mit einem Rand (130), der das Horn umgibt und mit dem Gehäuse verbunden ist, wobei das Horn und der Rand dazwischen einen Begrenzer (140) bestimmen, der die Ausbreitung der Ultraschallvibrationen zum Rand hin begrenzt.

21. Ultraschallabgebendes Hautpflegegerät nach Anspruch 20, wobei der Begrenzer durch einen Hohlraum bestimmt wird, der an der Grenze zwischen dem Horn und dem Rand ausgebildet ist.

22. Ultraschallabgebendes Hauptpflegegerät nach einem der Ansprüche 1 bis 8, ferner umfassend: eine Bewegungsdetektionsschaltung (50), die überwacht, ob sich die kombinierte Vibrationsmasse bewegt, und ein Bewegungsdetektionssignal liefert, wenn sich die Vibrationsmasse bewegt; wobei die Steuerschaltung die Treiberschaltung steuert, um den elektrischen Impuls zu beenden oder zu begrenzen, wenn das Lastdetektionssignal nicht innerhalb des vorbestimmten Zeitraums empfangen wird oder wenn das Bewegungsdetektionssignal nicht über eine kritische Zeitdauer kontinuierlich ist, selbst wenn das Lastdetektionssignal innerhalb des Zeitraums erkannt wird.

## Revendications

1. Dispositif pour faire pénétrer un agent actif pour la peau dans le corps humain via la peau en utilisant un appareil d'application d'ultrasons qui applique des ultrasons au corps humain via la peau, comprenant :
(1) une composition comprenant :
(a) de 0,001 % à 30 % de l'agent actif pour la peau ;
(b) un agent améliorant la viscosité qui fournit à la composition une viscosité allant de 1000 mPas à 1 000 000 mPas ;
(c) de 0,1 % à 30 % d'un humectant hydrosoluble ; et
(d) un véhicule aqueux ; dans lequel la composition est essentiellement dépourvue d'agents tensioactifs ; et
(2) l'appareil d'application d'ultrasons comprenant :
(e) une tête applicatrice (100) pour appliquer à la peau des ultrasons à une fréquence allant d'environ 3 MHz à 10 MHz et une intensité allant d'environ 0,1 W/cm² à 2 W/cm² ; et
(f) un circuit de commande (80) pour contrôler les conditions d'application de l'élément d'application,
dans lequel ledit appareil d'application d'ultrasons comprend en outre : un logement (10) pourvu de la tête applicatrice (100) qui applique des ultrasons sur la peau d'un utilisateur ; et un circuit de pilotage (20) qui donne une impulsion électrique pour actionner ladite tête applicatrice de façon à générer les ultrasons ; ladite tête applicatrice comprenant: un élément vibrant (110) générant les ultrasons, et une sonde (12) comportant une face de montage (121) et une face opposée à la peau (122) qui est adaptée en cours d'utilisation pour venir en contact avec la peau, ladite sonde portant ledit élément vibrant sur ladite face de montage pour transmettre lesdits ultrasons à la peau par ladite face opposée à la peau, ledit élément vibrant et ladite sonde étant intégrés dans une masse vibrante combinée qui résonne avec l'impulsion électrique d'une fréquence de résonance provenant dudit circuit de pilotage pour générer les ultrasons, ladite masse vibrante combinée définissant ledit élément d'application qui donne une première impédance électriquement équivalente lorsqu'elle est normalement chargée par contact avec la peau, et donne une deuxième impédance électriquement équivalente lorsqu'elle est déchargée, un circuit de détection de charge (40) qui est relié pour surveiller si ladite masse vibrante combinée donne la première ou deuxième impédance électriquement équivalente et fournit un signal de détection de charge uniquement lorsqu'on voit ladite première impédance électriquement équivalente, le circuit de commande (80) étant adapté pour limiter ou arrêter l'impulsion électrique lorsque le signal de détection de charge n'est pas reçu endéans une période de temps prédéterminée, ladite masse vibrante combinée ayant une structure qui limite les vibrations à une partie centrale de ladite masse vibrante combinée pour réduire une résonance parasite, en différenciant de ce fait ladite première impédance électriquement équivalente de ladite deuxième impédance électriquement équivalente pour une distinction entre elles, ledit circuit de commande constituant ledit élément de commande qui reçoit ladite première impédance électriquement équivalente afin de faire varier l'intensité des ultrasons générés au niveau dudit élément vibrant conformément à l'ordre de grandeur de ladite première impédance électriquement équivalente.

2. Dispositif selon la revendication 1, dans lequel l'agent actif pour la peau est choisi parmi les composés d'acide ascorbique, les composés de vitamine B3, et leurs mélanges.

3. Dispositif selon la revendication 1, dans lequel l'agent améliorant la viscosité comprend un copolymère acide carboxylique/carboxylate ; et un polymère dérivé de cellulose.

4. Dispositif selon la revendication 3, dans lequel l'agent améliorant la viscosité fournit une viscosité allant de 3000 mPas à 100 000 mPas.

5. Dispositif selon la revendication 1, dans lequel l'humectant hydrosoluble est choisi dans le groupe constitué de butylènes glycol, pentylène glycol, et leurs mélanges.

6. Dispositif selon la revendication 1, dans lequel la composition comprend au moins 70 % d'eau.

7. Dispositif selon la revendication 1, dans lequel la composition comprend, en outre, de 0,1 % à 15 % d'un composant huileux.

8. Dispositif selon la revendication 7, dans lequel le composant huileux est choisi dans le groupe constitué d'huiles hydrocarbure, esters d'acide gras, huiles de silicone, et leurs mélanges.

9. Dispositif selon l'une quelconque des revendications 1 à 8, dans lequel ledit élément vibrant comprend un élément piézoélectrique (110) sous la forme d'un disque circulaire ayant des faces d'extrémité supérieure et inférieure plates, et des électrodes supérieure et inférieure (111, 112) déposées respectivement sur lesdites faces d'extrémité supérieure et inférieure plates, et des électrodes supérieure et inférieure, ladite impulsion électrique étant appliquée à travers lesdites électrodes supérieure et inférieure.

10. Dispositif selon la revendication 9, dans lequel au moins l'un parmi ladite électrode supérieure, ladite électrode inférieure, et ledit élément piézoélectrique a une ouverture centrale (114) pour limiter les vibrations au centre de ladite masse vibrante combinée.

11. Dispositif selon la revendication 9, dans lequel chacun parmi ladite électrode supérieure, ladite électrode inférieure, et ledit élément piézoélectrique a une ouverture centrale pour limiter les vibrations au centre de ladite masse vibrante combinée.

12. Dispositif selon la revendication 10, dans lequel au moins l'une parmi ladite électrode supérieure et ladite électrode a un diamètre plus petit que celui dudit élément piézoélectrique pour laisser les parties périphériques de la face extrême correspondante dudit élément piézoélectrique non couvertes.

13. Dispositif selon la revendication 9, dans lequel au moins l'une parmi lesdites électrodes supérieure et inférieure est divisée par au moins une fente en une pluralité de segments identiques, ladite au moins une fente (116) s'étendant diamétralement pour laisser le centre et la partie de bande s'étendant diamétralement dudit élément piézoélectrique non couverts.

14. Dispositif selon la revendication 9, dans lequel au moins l'une parmi lesdites électrodes supérieure et inférieure a au moins une fente qui découvre la partie centrale dudit élément piézoélectrique.

15. Dispositif selon la revendication 9, dans lequel ladite sonde a un trou central pour limiter les vibrations au centre de ladite masse vibrante combinée.

16. Dispositif selon la revendication 10, dans lequel ladite sonde a un trou central (134) pour limiter les vibrations au centre de ladite masse vibrante combinée.

17. Dispositif selon la revendication 9, dans lequel ladite électrode supérieure est couverte sur son centre avec un élément élastique (150) absorbant les vibrations au centre de ladite masse vibrante combinée.

18. Dispositif selon la revendication 17, dans lequel ledit élément élastique est un caoutchouc de silicone.

19. Dispositif selon la revendication 9, dans lequel ladite électrode supérieure dudit élément piézoélectrique est couverte sur son centre avec une masse de soudure (160) pour une connexion électrique de l'électrode supérieure à un fil conducteur (101) venant dudit circuit de pilotage, la masse de soudure ajoutant un poids au centre de l'élément piézoélectrique.

20. Dispositif selon la revendication 1, dans lequel ladite sonde est formée en tant que partie intégrante de celle-ci avec un rebord (130) qui entoure ladite sonde et est reliée audit logement, ladite sonde et ledit rebord définissent entre eux un restricteur (140) qui empêche les vibrations des ultrasons de se propager vers ledit rebord.

21. Dispositif de soin de la peau applicateur d'ultrasons selon la revendication 20,
dans lequel ledit restricteur est défini par une cavité formée à la limite entre ladite sonde et ledit rebord.

22. Dispositif de soin de la peau applicateur d'ultrasons selon l'une quelconque des revendications 1 à 8, incluant en outre : un circuit de détection de mouvement (50) qui surveille si ladite masse vibrante combinée bouge et fournit un signal de détection de mouvement lorsque ladite masse vibrante bouge de la sorte ; ledit circuit de commande commandant ledit circuit de pilotage pour arrêter ou limiter ladite impulsion électrique lorsque ledit signal de détection de charge n'est pas reçu endéans ladite période de temps prédéterminée ou lorsque ledit signal de détection de mouvement n'est pas continu au cours d'une durée cruciale même en présence dudit signal de détection de charge qui est détecté endéans ladite période de temps.
